# EUROPEAN PATENT SPECIFICATION

(11) **EP 3 903 794 B1**
(45) Date of publication and mention of the grant of the patent: **24.07.2024**
(21) Application number: 19897870.2
(22) Date of filing: 04.12.2019
(51) Int. Cl.: A61K 35/28, C12N 15/06, A61P 37/06

(54) **PHARMACEUTICAL COMPOSITION COMPRISING CLONAL STEM CELLS FOR TREATING GRAFT-VERSUS-HOST DISEASE**
PHARMAZEUTISCHE ZUSAMMENSETZUNG MIT KLONALEN STAMMZELLEN ZUR BEHANDLUNG VON GRAFT-VERSUS-HOST-KRANKHEIT
COMPOSITION PHARMACEUTIQUE COMPRENANT DES CELLULES SOUCHES CLONALES POUR LE TRAITEMENT LA MALADIE DU GREFFON CONTRE L'HÔTE

(30) Priority: 17.12.2018 KR 20180163010
(43) Date of publication of application: 03.11.2021
(73) Proprietor: SCM Lifescience Co., Ltd., Jung-gu Incheon 22332 (KR)
(72) Inventor: SONG, Sun Uk, Incheon 22003 (KR); KIM, Si Na, Incheon 21997 (KR); MOON, Jeong Hyun, Incheon 21949 (KR)
(74) Representative: Altmann Stößel Dick Patentanwälte PartG mbB
(86) International application number: PCT/KR2019/017031
(87) International publication number: WO 2020/130431

(56) References cited:
- WO-A2-2008/002914
- KR-A- 20070 018 738
- KR-A- 20080 105 555
- KR-A- 20100 120 532
- KR-A- 20160 002 490
- KR-A- 20180 042 476
- US-A1- 2008 299 656
- MYUNG-SHIN JEON: "Characterization of mouse clonal mesenchymal stem cell lines established by subfractionation culturing method", WORLD JOURNAL OF STEM CELLS, vol. 3, no. 8, 1 January 2011 (2011-01-01), CN, pages 70, XP055352849, ISSN: 1948-0210, DOI: 10.4252/wjsc.v3.i8.70
- YOO HYUN SEUNG ET AL: "Mesenchymal Stem Cell Lines Isolated by Different Isolation Methods Show Variations in the Regulation of Graft-versus-host Disease", IMMUNE NETWORK, 1 August 2013 (2013-08-01), Korea (South), pages 133 - 140, XP055904922, Retrieved from the Internet <URL:https://immunenetwork.org/DOIx.php?id=10.4110/in.2013.13.4.133> [retrieved on 20220324], DOI: 10.4110/in.2013.13.4.133
- GU, HONGHOE: "Prevention and Treatment Effects of Improved Human Mesenchymal Stem Cells on Graft versus Host Disease", FINAL REPORT OF HEALTH & MEDICAL TECHNOLOGY R&D PROGRAM, SAMSUNG SEOUL HOSPITAL, KR, vol. A090729, 1 April 2011 (2011-04-01), KR , pages 1 - 79, XP009528664

## Description

### [Technical Field]

The present disclosure relates to a pharmaceutical composition for use in preventing or treatinggraft-versus-host disease, the pharmaceutical composition comprising monoclonal stem cells (MSCs) obtained by improved subfractionation culture of said monoclonal stem cells, and to a method of producing a pharmaceutical composition comprising monoclonal stem cells.

### [Background Art]

Graft-versus-host disease is a disease that might occur when T lymphocytes, which are immune cells of others, introduced into the host through treatment activities such as allogeneic bone marrow transplantation or peripheral blood hematopoietic stem cell transplantation, or blood transfusion that have been done for reasons of reduced immunity, and the like, are not killed by the host immune system, but are proliferated in the host if the host immune function has reduced, and then lymphocytes recognize the patient's cells as non-self and attack the host, and is a disease that can lead to death in severe cases depending on the severity of symptoms. The causes of graft-versus-host disease are largely divided into two types: weakened host immunity and blood transfusions from family. Graft-versus-host disease is divided into acute and chronic forms. If graft-versus-host disease occurs within 100 days after surgery, it is classified as being acute, and if graft-versus-host disease occurs after 100 days, it is classified as being chronic. Graft-versus-host disease mostly progresses to an acute form, and the T cells contained in the graft respond to specific antigens present in the host cell and cause various symptoms. The symptoms occurred include infection, pneumonia caused by cytomegalovirus, hepatitis, and gastroenteritis. As a skin lesion, a skin rash similar to measles occurs. It is different from other skin diseases because it mainly occurs on the auricles, palms, and soles. Along with the skin disease, liver disease is accompanied, so jaundice occurs, and liver somatic index increases. Problems with the gastrointestinal tract cause nausea, vomiting, loss of appetite, abdominal pain, and diarrhea.

Until now, there is no fundamental treatment method for graft-versus-host disease, and only treatments that can alleviate symptoms are possible, and steroid therapy is being used as an effective treatment.

Recently, attempts are being made to use stem cells for the treatment of various diseases. Stem cells have potential to grow into tissues of all 210 organs in our body, which can be infinitely divided and can be differentiated into desired organs by the suitable manipulation. Due to such characteristics, stem cells are in the spotlight as a new therapeutic agent. The treatability of incurable diseases using stem cells is very high, and thus it is expected to be able to treat numerous diseases such as leukemia, osteoporosis, hepatitis, Parkinson's disease, senile dementia, and burns.

WO2009/040666 discloses a therapeutic agent for treating acute or chronic graft-versus-host disease using clonal marrow stem cells (cMSCs) as active ingredient.

KR 2010 0120 532 discloses a method for reactivating multipotency and proliferation rate of aged adult stem cells.

WO 2008/002914 discloses a method of expanding mesenchymal stem cells ex vivo including the steps of seeding a collection of cells containing the mesenchymal stem cells on a substrate so that a low density of mesenchymal stem cells adhere to the substrate; expanding the adhered mesenchymal stem cells on the substrate; removing the expanded mesenchymal stem cells from the substrate; and reseeding the mesenchymal stem cells on the same or different substrate.

However, stem cells still have many limitations in that it is difficult to obtain stem cells in large quantities. Although the method of obtaining stem cells from frozen embryonic cells may be effective, there is still a lot of controversy in terms of ethics. In order to address these issues, many studies have also been conducted on a method of obtaining stem cells using adult stem cells or a somatic cell nucleus transplantation method. The field that is more actively conducted than research on embryonic stem cells is adult stem cell research. Adult stem cells are cells that remain in various organs such as the central nervous system or bone marrow and participate in the organ development during the growth period and the regeneration of damage. Since adult stem cells are present in various organs, they can be obtained from various sites including bone marrow, spleen, adipocytes, and the like, but the method obtained from bone marrow is most commonly practiced. However, it is difficult to obtain uniformly shaped cells at all times while mesenchymal stem cells among many kinds of bone marrow cells are isolated and cultured. Thus, studies are conducted to address these issues.

The present inventors have invented a method for isolating stem cells named as a novel subfractionation culturing method, and filed a patent application under Korean Patent Application No. 10-2006-0075676, which was granted for patent. The subfractionation culturing method may be performed at a lower cost than other methods. Moreover, there is no contamination issue, and clonal mesenchymal stem cells (cMSCs) can be obtained effectively without risk to mix other stem cells. Therefore, the subfractionation culturing method shows the unexpected superiority compared to the other methods of obtaining stem cells. However, despite the superiority of the method, the subfractionation culturing method has limitations in which it is difficult to rapidly obtain the monoclonal mesenchymal stem cell group. These are because in order to mass-produce mesenchymal stem cells to be used as a final product, the method requires production of a working cell bank which is used for carrying out the process of obtaining the final product, thereby obtaining a sufficient amount of mesenchymal stem cells and needs the culture with at least passage 10.

Accordingly, the development of therapeutic agents using stem cells still has various technical limitations, and in particular, there is no known stem cell producing method for effectively treating graft-versus-host disease and a method of treating graft-versus-host disease using the same.

### [Disclosure]

### [Technical Problem]

While conducting research to induce rapid proliferation of stem cells through the improvement of the subfractionation culturing method as described above, the present inventors identified that when an improved subfractionation culturing method in which culture cell density is adjusted low and an antioxidant is added was used, it was possible to induce an increase in the effective cell proliferation rate only with a few subcultures, and that the monoclonal mesenchymal stem cells obtained therethrough increased the therapeutic effect of graft-versus-host disease compared to the conventional stem cells or the clonal mesenchymal stem cells obtained from the conventional subfractionation culturing method, and then completed the present disclosure.

Accordingly, an aspect of the present disclosure is directed to providing a pharmaceutical composition for preventing or treatinggraft-versus-host disease, the pharmaceutical composition comprising monoclonal stem cells (MSCs) obtained through a method of subfractionation culture and proliferation of said monoclonal stem cells that has improved the conventional subfractionation culturing method, and a method of producing a pharmaceutical composition comprising monoclonal stem cells .

### [Technical Solution]

An exemplary embodiment of the present disclosure provides a pharmaceutical composition for use in preventing or treating graft-versus-host disease, the pharmaceutical composition comprising monoclonal stem cells obtained by steps of 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 13, wherein the medium of step 5) and 6) is supplemented with an antioxidant.

In addition, an exemplary embodiment of the present disclosure provides a method of producing a pharmaceutical composition comprising monoclonal stem cells, the method comprising steps of 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 13, wherein the medium of step 5) and 6) is supplemented with an antioxidant

### [Advantageous Effects]

According to an improved method of subfractionation culture and proliferation of stem cells of the present disclosure, it is possible to obtain a large quantity of desired monoclonal mesenchymal stem cells in a short time by rapid proliferation of monoclonal mesenchymal stem cells, and the monoclonal mesenchymal stem cells obtained thereby are stem cells with enhanced therapeutic effects on graft-versus-host disease, and thus may be beneficially used as a graft-versus-host disease therapeutic agent.

### [Description of Drawings]

FIG. 1 is a view showing a subfractionation culturing method for isolating monoclonal mesenchymal stem cells from human bone marrow.
FIG. 2 is a view showing the results of identifying the morphological changes in monoclonal mesenchymal stem cells according to cell culture density and cell subculture through microscopic observation.
FIG. 3 is a view showing the results of identifying the changes in cell size and granularity of monoclonal mesenchymal stem cells according to cell culture density and cell subculture by an average value of forward-scattered (FSC) (A) light and side-scattered (SSC) (B) light through flow cytometry (FACS) analysis (*p < 0.05, **p < 0.01, ***p < 0.005).
FIG. 4 is a view showing the results of identifying whether cells are aged after staining monoclonal mesenchymal stem cells by beta galactosidase (beta gal) activity in which cell culture densities and cell subcultures vary.
FIG. 5 is a view showing the results of identifying the aging-related genes p15 and p16 and the proliferation marker PCNA by RT-PCR after culturing the monoclonal mesenchymal stem cells of passage 15 (P15) while the cell culture density varies.
FIG. 6 is a view showing the results of identifying the proliferation capacity of monoclonal mesenchymal stem cells through population doubling time (PDT) and population doubling level (PDL) according to cell culture density and cell subculture (*p < 0.05, **p < 0.01, ***p < 0.005).

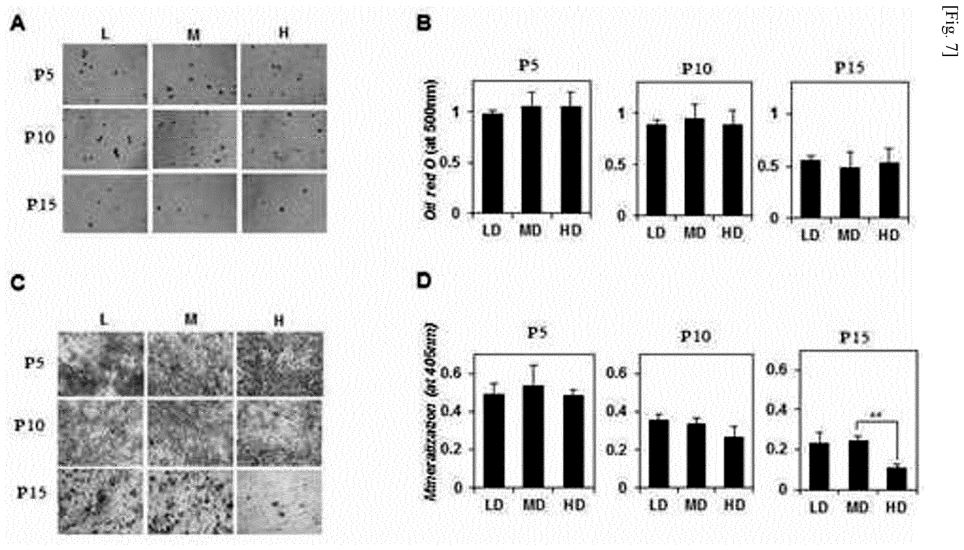

FIG. 7 is a view showing the results of identifying the differentiation ability of monoclonal mesenchymal stem cells according to cell culture density and cell subculture (*p < 0.05, **p <0.01, ***p < 0.005). FIG. 7A is a result of identifying the differentiation potential of monoclonal mesenchymal stem cells into adipocytes through Oil red O histological staining according to cell culture and cell subculture. FIG. 7B is a view showing quantification of the degree of histological staining of FIG. 7A. FIG. 7C is a result of identifying the differentiation potential of monoclonal mesenchymal stem cells into ossified cells through Alizarin red S histological staining according to cell culture and cell subculture. FIG. 7D is a view showing quantification of the degree of histological staining of FIG. 7C.
FIG. 8 is a view showing the results of total reactive oxygen species (ROS) production of monoclonal mesenchymal stem cells according to cell culture density and cell subculture (A) and identifying DNA damage accordingly through comet assay (B) (*p < 0.05, **p < 0.01, ***p < 0.005).
FIG. 9 is a view showing the results of measuring the concentration of 8-oxo-deoxyguanosine (8-hydroxy-2'-deoxyguanosine; 8-OHdG) in order to identify the degree of DNA damage caused by reactive oxygen species produced according to cell culture density and cell subculture (*p < 0.05, **p < 0.01, ***p < 0.005).
FIG. 10 is a view showing the results of identifying the changes in the proliferation capacity of cells obtained by culturing monoclonal mesenchymal stem cells of passages 11 (P1 1) to 15 (P15) under the condition of only high-density (HD) or high-density + ascorbic acid (a kind of antioxidant) addition (HD + AA).
FIG. 11 is a view showing the results of comparing the level of reactive oxygen species produced after culturing monoclonal mesenchymal stem cells of passage 15 (P15) under the condition of only high-density (HD) or high-density conditions + ascorbic acid addition (HD + AA) (*p < 0.05, **p < 0.01, ***p < 0.005).
FIG. 12A is a diagram illustrating a comparison of the conventional subfractionation culturing method and the improved subfractionation culturing method.
FIG. 12B is a schematic view of an improved subfractionation culturing method, and is a schematic view illustrating a low-density culture corresponding to the culture at passage 2 or later that is different from the conventional subfractionation culturing method.

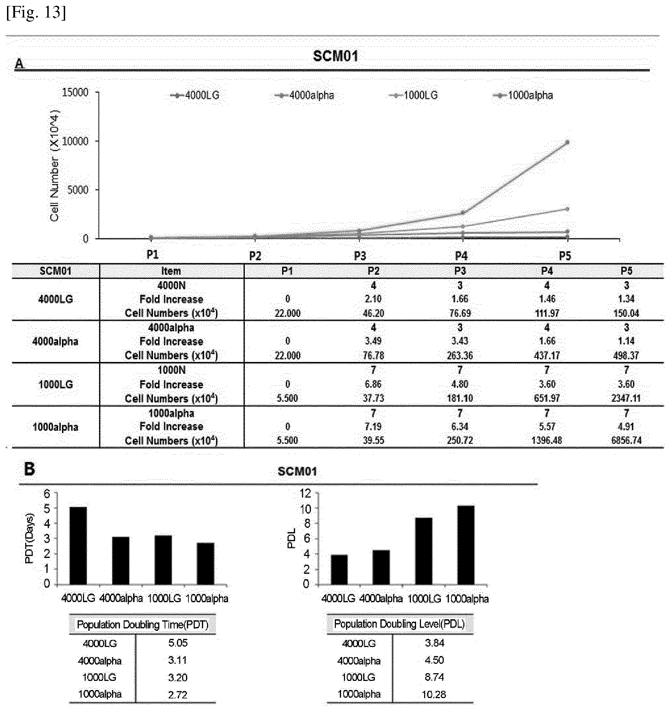

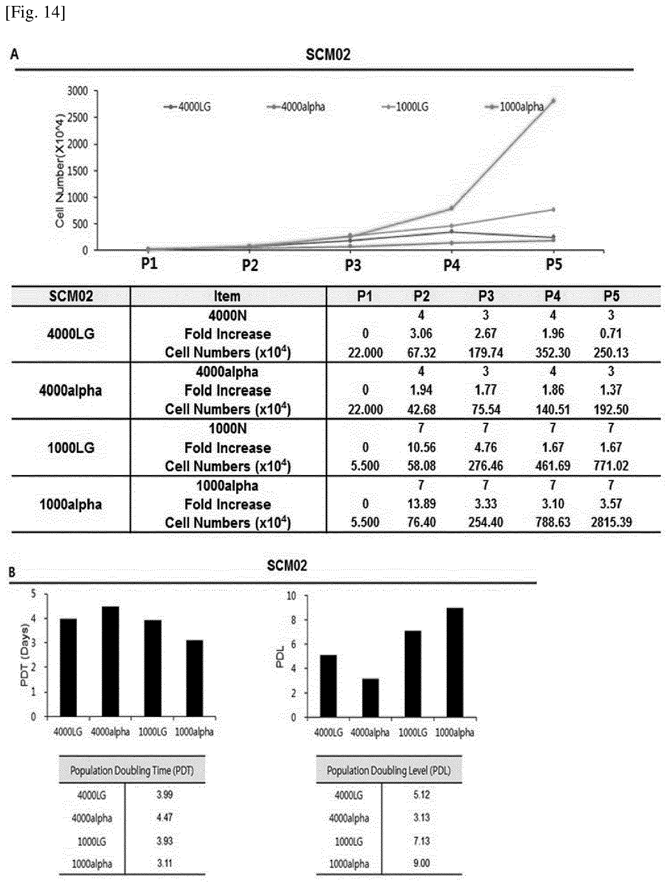

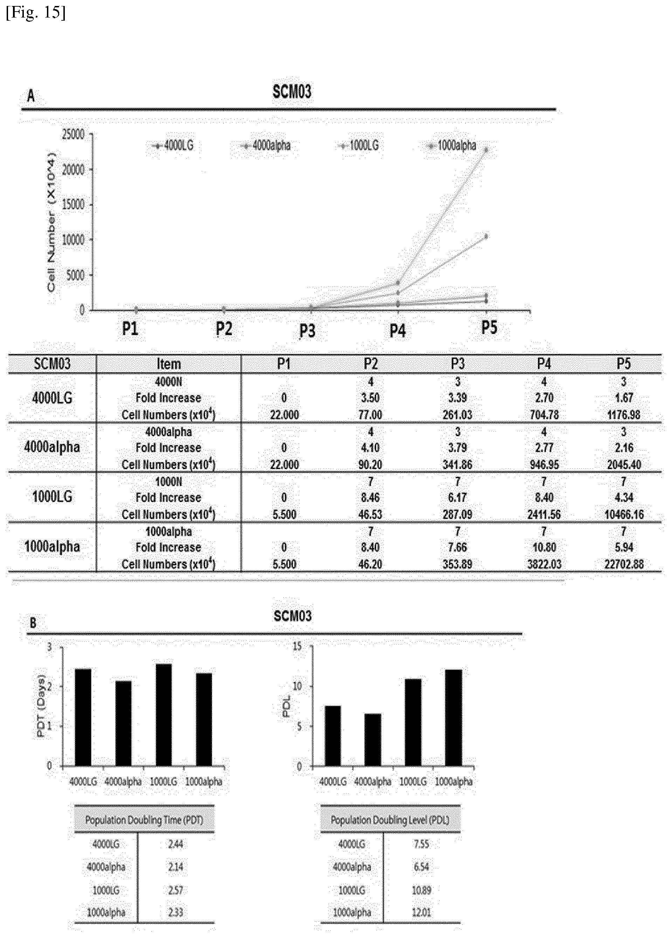

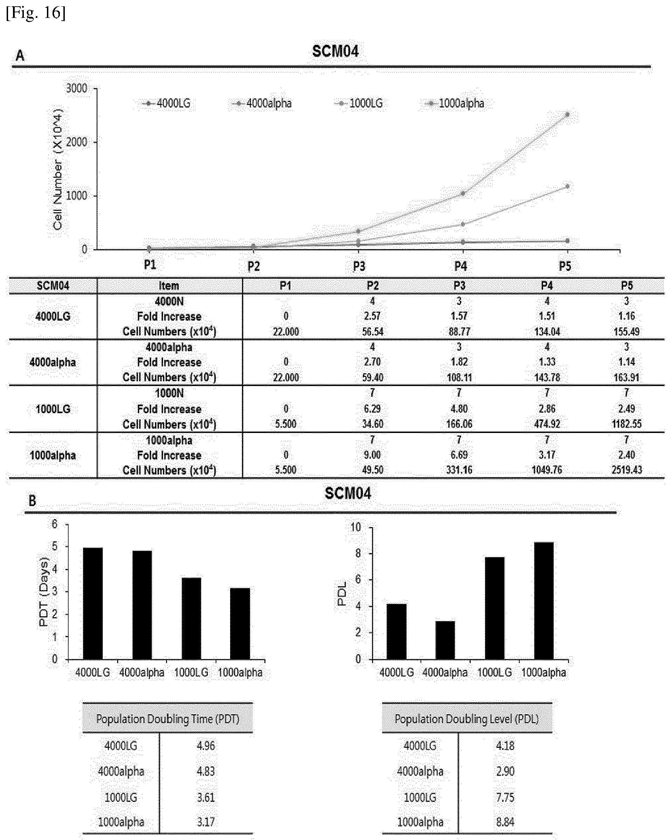

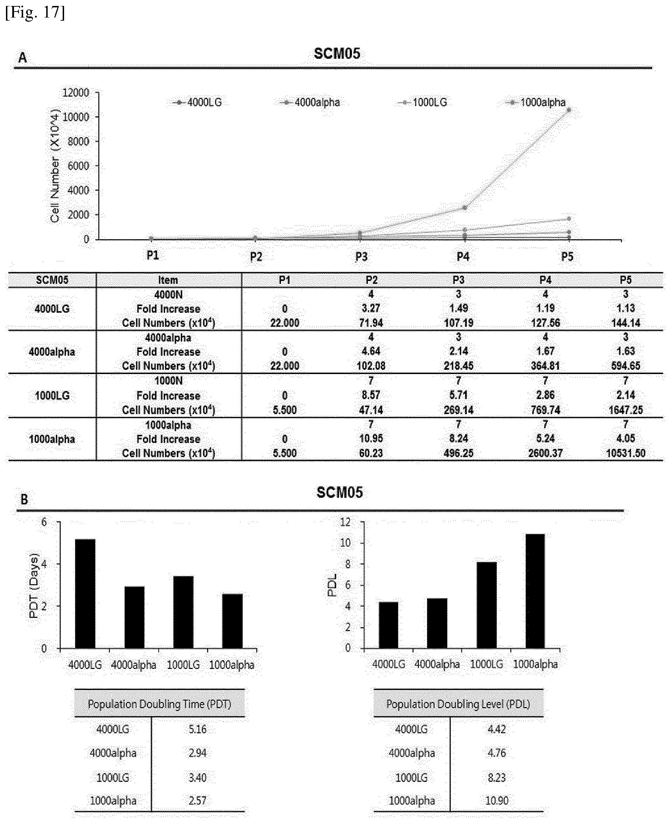

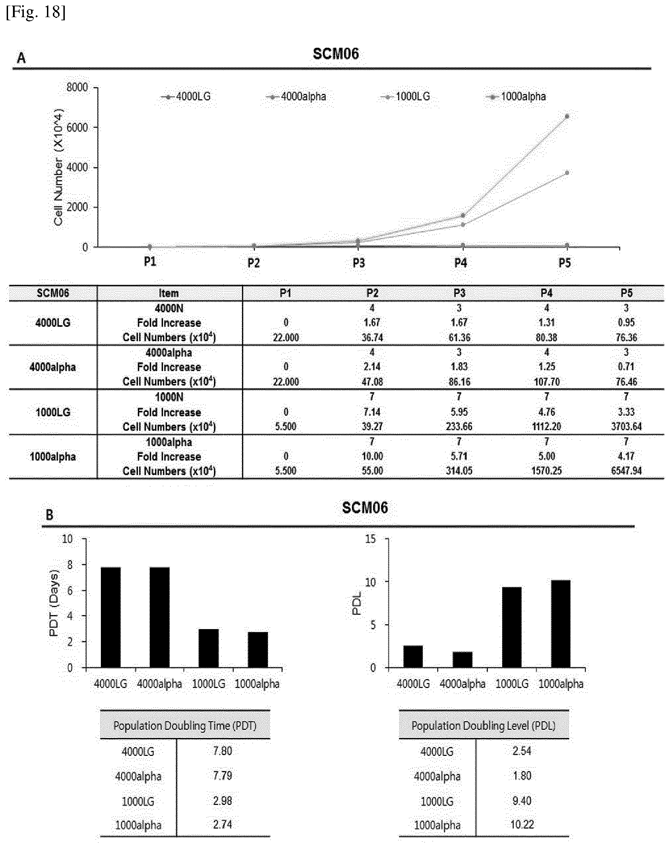

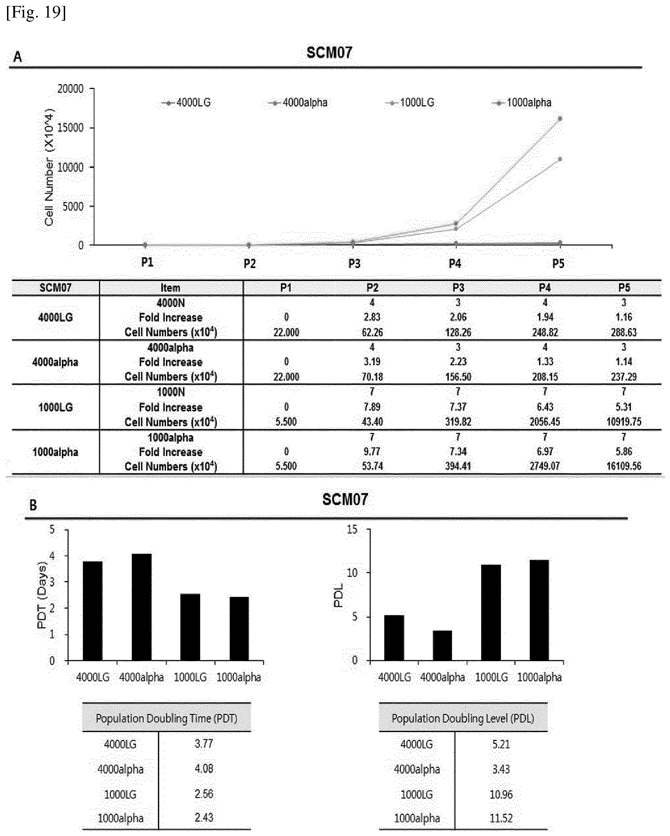

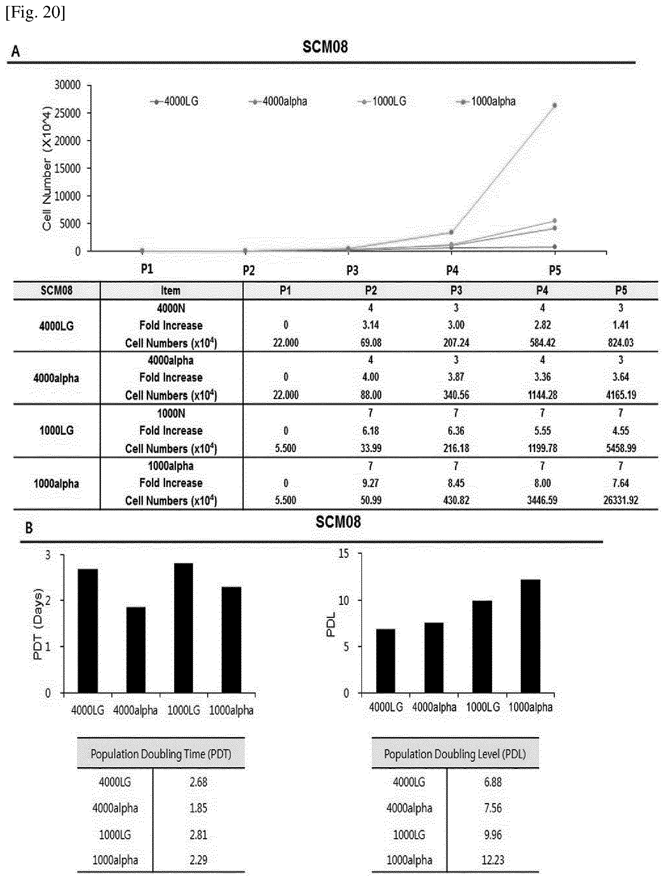

FIGS. 13 to 20 are views showing the results of identifying proliferation rate of cells in which SCM01 to SCM08 monoclonal mesenchymal stem cells obtained by the subfractionation culturing method are inoculated at a density of 1,000 or 4,000 cells/cm², and the cells are cultured using LG-DMEM (Dulbecco's Modified Eagle Medium, low glucose) and α-MEM (Minimum Essential Medium α) culture media with or without an antioxidant. A in each view shows the changes in the number of cells according to passage 1 (P1) to passage 5 (P5) of each experimental group, and B in each diagram shows the results of PDT and PDL of each experimental group.
FIG. 21 is a view showing the results of identifying the cell proliferation rate of an experimental group in which a LG-DMEM medium without an antioxidant is used, and only the cell density varies at a density of 1,000 or 4,000 cells/cm².
FIG. 22 is a view showing the results of identifying PDT and PDL of an experimental group in which a LG-DMEM medium without an antioxidant is used, and only the cell density varies at a density of 1,000 or 4,000 cells/cm².
FIG. 23 is a view showing the results of identifying the cell proliferation rate of an experiment group in which a α-MEM medium with an antioxidant is used, and only the cell density varies at a density of 1,000 or 4,000 cells/cm².
FIG. 24 is a view showing the results of identifying PDT and PDL of an experimental group in which a α-MEM medium with an antioxidant is used, and only the cell density varies at a density of 1,000 or 4,000 cells/cm².
FIG. 25 is a view showing the results of identifying the cell proliferation rate of an experimental group in which the cell density is fixed at a density of 1,000 cells/cm² and the culture medium is LG-DMEM or α-MEM.
FIG. 26 is a view showing the results of identifying PDT and PDL of an experimental group in which the cell density is fixed at a density of 1,000 cells/cm² and the culture medium is LG-DMEM or α-MEM.
FIG. 27 is a schematic view showing a method of producing an animal model of graft-versus-host disease.
FIG. 28 is a view identifying the expression of clinical symptoms in the produced animal model of graft-versus-host disease (A graft-versus-host disease-causing mice).
FIG. 29 is a view showing the results of identifying the MHC II phenotype in the blood of C57BL/6 recipients through flow cytometry in mice induced with graft-versus-host disease.
FIG. 30 is a view showing the results of identifying the skin layer of the mice on the 7th and 14th days of induction of graft-versus-host disease through tissue staining.
FIG. 31 is a view showing the results of identifying the change in the weight of the mice for 4 weeks after induction of graft-versus-host disease.
FIG. 32 is a schematic view of an experiment to identify the treatment effect after preparation of an animal model of graft-versus-host disease.
FIG. 33 is a schematic view of an experiment to identify the treatment effect of graft-versus-host disease using cMSC1 of the improved subfractionation culturing method.
FIG. 34 is a view showing the results of identifying the survival rate of graft-versus-host disease mice according to the administration of cMSC1 of the improved subfractionation culturing method.
FIG. 35 is a view showing the results of identifying the effect of improving clinical symptoms of graft-versus-host disease according to the administration of cMSC 1 of the improved subfractionation culturing method.
FIG. 36 is a view showing the results of identifying the effect of improving skin symptoms among the clinical symptoms of graft-versus-host disease according to the administration of cMSC1 of the improved subfractionation culturing method.
FIG. 37 is a view showing the results of identifying the effect of improving fur texture symptoms among the clinical symptoms of graft-versus-host disease according to the administration of cMSC1 of the improved subfractionation culturing method.
FIG. 38 is a view showing the results of identifying the effect of improving diarrhea symptoms among the clinical symptoms of graft-versus-host disease according to the administration of cMSC1 of the improved subfractionation culturing method.
FIG. 39 is a view showing the results of identifying the effect of improving ascites symptoms among the clinical symptoms of graft-versus-host disease according to the administration of cMSC 1 of the improved subfractionation culturing method.
FIG. 40 is a view showing the results of identifying the effect of improving posture among the clinical symptoms of graft-versus-host disease according to the administration of cMSC1 of the improved subfractionation culturing method.
FIG. 41 is a view showing the results of identifying the number of cells and cell sizes of the conventional subfractionation culturing method cMSC2 and the improved subfractionation culturing method cMSC1 with a microscope.
FIG. 42 is a view showing the results of identifying the cell sizes of the conventional subfractionation culturing method cMSC2 and the improved subfractionation culturing method cMSC1 through a Nucleo Counter NC-250 device.
FIG. 43 is a view showing the results of identifying the cell sizes of the conventional subfractionation culturing method cMSC2 and the improved subfractionation culturing method cMSC1 through flow cytometry.
FIG. 44 is a view showing the results of identifying the expression levels of immune-related markers of the conventional subfractionation culturing method cMSC2 and the improved subfractionation culturing method cMSC1 through qPCR.
FIG. 45 is a view showing the results of comparing the survival rate according to the conventional subfractionation culturing method cMSC2 and the improved subfractionation culturing method cMSC1 (100) (100 cells/cm² culture), cMSC1 (1000) (1,000 cells/cm² culture) treatments in the GVHD animal model.
FIG. 46 is a view showing the results of comparing the clinical symptom improvement effect according to the conventional subfractionation culturing method cMSC2 and the improved subfractionation culturing method cMSC1 (100) (100 cells/cm² culture), cMSC1 (1000) (1,000 cells/cm² culture) treatments in the GVHD animal model.

### [Modes of the Invention]

The present disclosure is directed to a pharmaceutical composition for use in preventing or treating graft-versus-host disease, the pharmaceutical composition comprising monoclonal stem cells (cMSC1) obtained through an improved method of subfractionation culture and proliferation of mesenchymal stem cells.

In addition, the present disclosure is directed to a method of producing a pharmaceutical composition comprising monoclonal stem cells obtained through an improved method of subfractionation culture and proliferation of mesenchymal stem cells.

In addition to the advantages of the subfractionation culturing method that may obtain stem cells quickly and without contamination, the monoclonal stem cells, which are active ingredients of the present disclosure, are stem cells obtained through an improved subfractionation culturing method capable of obtaining a large quantity of desired monoclonal stem cells in a short time without the need for a Working Cell Bank (WCB) production process by rapid proliferation of monoclonal stem cells, preferably monoclonal mesenchymal stem cells. The monoclonal stem cells obtained through the above method are stem cells with enhanced therapeutic effects on graft-versus-host disease compared to the stem cells obtained through the conventional subfractionation culturing method.

Hereinafter, the present disclosure will be described in detail.

An exemplary embodiment of the present disclosure provides a pharmaceutical composition for use in preventing or treating graft-versus-host disease, including monoclonal stem cells obtained by steps of 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 13, wherein the medium of step 5) and 6) is supplemented with an antioxidant.

The culture in steps 2) and 3) is performed at 30 to 40°C for 4 hours or less, preferably for 1 to 3 hours, more preferably for 1 hour 30 minutes to 2 hours 30 minutes. The repeated culture is performed at 30 to 40°C for 4 hours or less (preferably for 1 to 3 hours, more preferably for 1 hour 30 minutes to 2 hours 30 minutes) then is performed at 30 to 40°C for 12 to 36 hours (preferably for 18 to 30 hours) to be repeated 2 or 3 times. Subsequently, the culture is performed at 30 to 40°C for 24 to 72 hours and 36 to 60 hours (preferably for 36 to 60 hours). Each supernatant may be transferred to a new culture vessel to perform the next experiment.

According to one embodiment of the present disclosure, a brief summary of the isolation method is as follows.

Collection of bone marrow → Culture at 37°C for 2 hours → Culture at 37°C for 2 hours → Culture at 37°C for 2 hours → Culture at 37°C for 24 hours →
Transfer of only supernatant into new vessel
Transfer of only supernatant into new culture vessel coated with or without collagen (1)
Same as (1)
Same as (1)

Culture at 37°C for 24 hours → Culture at 37°C for 48 hours → Identification of growth on bottom of culture vessel → Culture at 37°C for 3 to 5 days → Identification of single cell group formation
Same as (1)
Same as (1)

The cultured cells form monoclonal cell groups. These monoclonal cell groups may be isolated and then subcultured. The present disclosure includes step 5) and step 6) of culture in order to rapidly obtain the monoclonal stem cells in large quantities obtained through the subfractionation culture, and to obtain the monoclonal stem cells with enhanced therapeutic effects on graft-versus-host disease.

The step 5) and step 6) is a process of inoculating and culturing monoclonal stem cells into a medium at a cell density of 1,000 cells/cm² or less, preferably 50 to 1,000 cells/cm². The present disclosure may induce rapid proliferation of monoclonal stem cells so that a final product may be obtained rapidly. Further, a master cell bank (MCB) may be prepared through a small number of subcultures.

The term "subfractionation culturing method" used herein refers to a method of isolating stem cells according to specific gravity, indicating a process in that first, human bone marrow is extracted and cultured in a cell culture medium, then, only a supernatant is obtained, transferred to a culture vessel with or without treatment of a coating agent, and cultured, and then the same processes are repeated several times. Such a subfractionation culturing method is characterized by repeatedly obtaining and culturing the supernatant without centrifugation. It is advantageous that monoclonal stem cells, preferably monoclonal mesenchymal stem cells may be obtained without contamination of other cells finally.

Steps 1) to 4) of steps 1) to 6) of the present disclosure may be performed in the same or equivalent to the subfractionation culturing method described in Korean Patent Application No. 10-2006-0075676, or Korean Patent Application No. 10-2007-0053298, and Korean Patent Application No. 10-2006-0075676.

Conventionally, Korean Patent Application No. 10-2007-0053298 discloses a method of obtaining cells in connection with the treatment of graft-versus-host disease, including the steps of: 1) collecting bone marrow from a subject; 2) culturing the collected bone marrow; 3) transferring only a supernatant of step 2) to a new vessel and culturing the supernatant; and 4) isolating only the supernatant of step 3) and repeatedly culturing the supernatant in a culture vessel with or without treatment of a coating agent. The method is to obtain monoclonal stem cells only with a difference in density without centrifugation, and thus uses the conventional subfractionation culturing method of Korean Patent Application No. 10-2006-0075676.

However, the subfractionation culturing method of Korean Patent Application No. 10-2006-0075676 and Korean Patent Application No. 10-2007-0053298 does not disclose a method of effectively obtaining monoclonal stem cells at a low passage, thereby obtaining monoclonal stem cells with remarkably improved therapeutic effects on graft-versus-host disease.

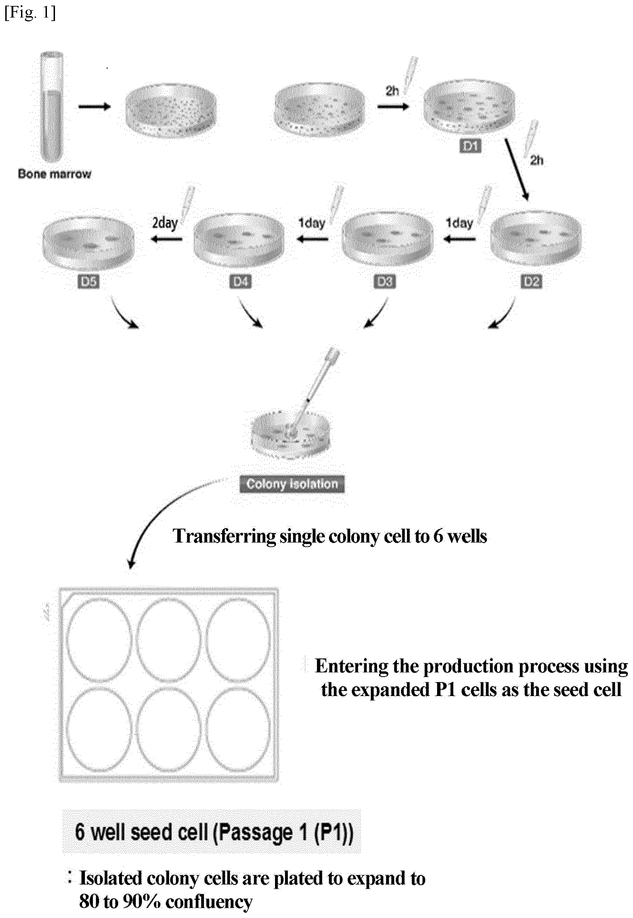

In the conventional subfractionation culturing method, as identified in FIG. 1, all cells obtained from a single colony were transferred to a 6-well plate and proliferated at 80-90% confluence, and then in order to obtain many cells without recognition of density control by using the proliferated passage 1 (P1) cells as seed cells, high-density culture was performed at 4,000 cells/cm².

The present disclosure relates to an "improved subfractionation culturing method" based on the fact that stem cells with excellent effects of preventing ortreating graft-versus-host disease may be effectively obtained by controlling the cell density in culture after passage 2, in which it is characterized in that the culturing after the seed cell is different from the conventional subfractionation culturing method. For example, specifically, the method includes the step of 5) inoculating the monoclonal stem cells of step 4) in a medium at a cell density of 50 to 1,000 cells/cm² to culture the cells at passage 1. The improved subfractionation culturing method may induce rapid proliferation of monoclonal stem cells compared to the conventional subfractionation culturing method, and thus the final product may be obtained quickly. The monoclonal stem cells for use in the treatment of graft-versus-host disease may be obtained through the culture of passage 2 to passage 13, and may obtain a sufficient amount of stem cells with excellent therapeutic effects on graft-versus-host disease only with a low subculture of less than passage 10 such as P2 to P8. For example, specifically, the method includes the step of 6) inoculating the subcultured monoclonal stem cells of step 5) in a medium at a cell density of 50 to 1000 cells/cm² to culture the cells at passages 2 to 13. For example, in the case of acute graft-versus-host disease, frozen stem cells may be used. The stem cells obtained through a culture of passage 8 or less may be frozen and thawed for use in the improved subfractionation culturing method of the present disclosure. In addition, in the case of chronic graft-versus-host disease requiring rapid treatment, fresh stem cells may be used for effective treatment. In this case, stem cells may be obtained by expanding the passage up to passage 13 to secure a larger amount of stem cells. The stem cells thus obtained are monoclonal stem cells with increased expression of immune-related markers, maintenance of differentiation potential, and suppression of aging compared to stem cells obtained by high-density culture, and are stem cells with remarkably improved therapeutic effects on graft-versus-host disease compared to the stem cells obtained through the conventional centrifugation methods and the stem cells obtained through the conventional subfractionation culturing method.

When the monoclonal stem cells of the present disclosure are cultured at a high density of 4,000 cells/cm² as in the conventional process, the cell proliferation ability may be markedly decreased, the markers of the mesenchymal stem cells may be changed, and the differentiation potential of stem cells may be eliminated. Thus, the monoclonal stem cells obtained through the improved subfractionation culturing method may be cultured at a low density that is, at the cell density of 50 to 1,000 cells/cm².

When monoclonal mesenchymal stem cells are cultured at a cell density of 1,000 cells/cm² or less, the cell proliferation ability is maintained remarkably high over an extended period of culture compared with mesenchymal stem cells cultured at a high density of 4,000 cells/cm². Thus, there is an advantage in that a large amount of monoclonal cells which is desired may be rapidly obtained without repeating a large number of passages. Further, when the monoclonal mesenchymal stem cells are cultured at the above-mentioned cell density, the cells have the advantage that their DNA may be less damaged, and they are less aged, thereby effectively maintaining the differentiation potential of the stem cells. Thus, monoclonal mesenchymal stem cells may be rapidly and quickly obtained to have excellent stem cell properties.

The medium used in the present disclosure may include a medium supplemented with an antioxidant or a medium containing an antioxidant.

An antioxidant may further be added to the medium to perform the culture. α-MEM containing an antioxidant can be used to perform the culture.

The antioxidants may include, without limitation, antioxidants that can be used in cell cultures. They may include one or more selected from the group consisting of glutathione, cysteine, cysteamine, ubiquinol, beta-mercaptoethanol and ascorbic acid (AA). When the medium is supplemented with an antioxidant, the antioxidant may be added at a concentration of 10 to 50 µg/ml, preferably 10 to 30 µg/ml, and more preferably 25 µg/ml.

In one embodiment of the present disclosure, α-MEM is used as a medium containing ascorbic acid as an antioxidant.

When a medium with an antioxidant is used as a culture medium of the present disclosure, the culture medium may be added with gentamicin as an antibiotic.

According to the method of the present disclosure, since monoclonal stem cells may be proliferated very effectively, a process of producing WCB using MCB may be omitted. This is a simplification of the process compared to the conventional subfractionation culturing method that needs to involve a process of producing WCB after MCB production.

The mesenchymal stem cells obtained through the method of the present disclosure may be stem cells obtained after passages 2 to 13, preferably after passages 2 to 12, and more preferably after passages 2 to 9. According to the improved subfractionation culturing method of the present disclosure, it is possible to sufficiently obtain mesenchymal stem cells with excellent therapeutic effects on graft-versus-host disease sufficient to be used as a therapeutic agent only with culture of less than passage 10. When more stem cells are needed quickly, such as chronic graft-versus-host disease, the passage may be increased to a maximum of passage 13, for example, passage 12 for culture to obtain more monoclonal stem cells with enhanced therapeutic effects on graft-versus-host disease.

They are stem cells obtained at a lower passage than the conventional process in which culture of at least passage 10 is essentially required, and by controlling the cell inoculation density, it is possible to easily obtain a large amount of mesenchymal stem cells rapidly proliferated in the low passage. At the same time, this demonstrates that they are mesenchymal stem cells with improved therapeutic effects on graft-versus-host disease.

In the present disclosure, the monoclonal stem cells (hereinafter referred to as "cMSC1") obtained by the improved subfractionation culturing method as described above with low density and antioxidant conditions, preferably 1,000 cells/cm² or less, are smaller in cell size and may be formed homogeneously compared to the monoclonal stem cells (hereinafter referred to as "cMSC2") obtained by the conventional subfractionation culturing method, and are stem cells capable of increasing the survival rate of acute chronic graft-versus-host disease as well as chronic graft-versus-host disease, increasing the expression of immune-related markers, and effectively improving the clinical factors of various graft-versus-host disease when administered to a subject.

The term "graft-versus-host disease" used herein refers to a disease in which an immune response occurs when the patient's body recognizes the T lymphocytes in the bone marrow or peripheral blood of a donor injected during allograft as foreign, and includes either chronic or acute graft-versus-host disease. Stem cells used in the treatment of graft-versus-host disease may be either fresh or frozen type, and may be appropriately selected and used according to clinical judgment.

Accordingly, in the present disclosure, the graft-versus-host disease may include, without limitation, either acute graft-versus-host disease or chronic graft-versus-host disease.

The monoclonal stem cells obtained by the improved subfractionation culturing method of the present disclosure have increased expression levels of immune-related markers compared to the stem cells obtained by the conventional subfractionation culturing method or centrifugation method. For example, the expression level of one or more immune-related markers selected from the group consisting of IDO (Indoleamine 2,3-dioxygenase), ICOSL (Induced T cell co-stimulator ligand), TSG6 (Tumor necrosis factor-inducible gene 6 protein), CXCL9 (chemokine (C-X-C motif) ligand 9), CXCL10 (chemokine (C-X-C motif) ligand 10), and HO-1 (Heme oxygenase-1) may be increased.

The pharmaceutical composition for use of the present disclosure may be prepared by including at least one pharmaceutically acceptable carrier in addition to the active ingredient for administration. The pharmaceutically acceptable carrier included in the pharmaceutical composition for use of the present disclosure is commonly used in preparation, and includes lactose, dextrose, sucrose, sorbitol, mannitol, starch, gum acacia, calcium phosphate, alginates, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but is not limited thereto. In addition to the above ingredients, the pharmaceutical composition for use of the present disclosure may further include a lubricant, a wetting agent, a sweetener, a flavor enhancer, an emulsifying agent, a suspension agent, a preservative, and the like.

A dose of the pharmaceutical composition for use of the present disclosure may vary depending on the formulation method, administration manner, administration schedule and/or administration route of the pharmaceutical composition, and may vary according to various factors including the type and degree of the reaction to be achieved via administration of the pharmaceutical composition, the type, age, weight, general health conditions, the symptoms or severity of diseases, gender, diet, and excretion of subject to which the composition is administrated, drugs used concurrently in the subject or in combination for transplantation, and ingredients of other compositions, and similar factors well known in the medical field. The effective dose may be easily determined and prescribed for desired treatment by those of ordinary skill in the art.

The dose of the pharmaceutical composition for use of the present disclosure may be, for example, 1 mg/kg to 1,000 mg/kg per day, but the dose is not intended to limit the scope of the present disclosure in any way.

The administration route and administration manner of the pharmaceutical composition for use of the present disclosure may be independent of each other, the administration method is not particularly limited, and the administration route and the administration manner may be an arbitrary administration route and administration route as long as they enable the pharmaceutical composition to reach the desired corresponding site.

The pharmaceutical composition may be administered orally or parenterally. The parenteral administration may be, for example, intravenous administration, intraperitoneal administration, intramuscular administration, transdermal administration, or subcutaneous administration, and the pharmaceutical composition may be applied or sprayed on a disease site, or inhaled, but are not limited thereto.

The term "subject" used herein includes a subject in need of preventing or treating graft-versus-host disease, and may be a mammal or a mammal other than a human.

An exemplary embodiment of the present disclosure provides a method of producing a pharmaceutical composition comprising monoclonal stem cells, the method comprising steps of 1) culturing bone marrow isolated from a subject in a first vessel; 2) transferring only a supernatant from the first vessel to a new vessel and culturing the supernatant; 3) culturing cells present in the new vessel and obtaining a supernatant; 4) obtaining monoclonal stem cells by repeating steps 2) and 3) at least once using the supernatant of step 3) as the supernatant of the first vessel of step 2); 5) inoculating the monoclonal stem cells of step 4) in a medium at a cell density of 50 to 1,000 cells/cm² to culture the cells at passage 1; and 6) inoculating the subcultured monoclonal stem cells of step 5) in a medium at a cell density of 50 to 1000 cells/cm² to culture the cells at passages 2 to 13, wherein the medium of step 5) and 6) is supplemented with an antioxidant.

In the production method of the present disclosure, the culture of step 5) or step 6) above is performed by inoculating the monoclonal stem cells in a medium at a cell density of 1,000 cells/cm².

In addition, in the production method of the present disclosure, the medium in step 5) and step 6) is a medium supplemented with an antioxidant.

According to the present disclosure, the monoclonal stem cells exhibiting excellent graft-versus-host disease prevention or treatment effect compared to the monoclonal stem cells obtained by the conventional subfractionation culturing method may be obtained rapidly and easily without WCB production.

In the production method of the present disclosure, the contents described in the composition above may be equally applied, and overlapping contents are omitted in order to avoid the complexity of the description.

Hereinafter, the present disclosure is described in detail with the examples.

### <Example 1> Establishment of Improved Subfractionation Culturing Method

In order to produce the monoclonal mesenchymal stem cells exhibiting superior effects on graft-versus-host disease, an improved method of subfractionation culture and proliferation of mesenchymal stem cells was used. The improved method of subfractionation culture and proliferation of mesenchymal stem cells varies the cell density and culture medium in the culture conditions of the subfractionation culturing method disclosed in Korean Patent Application No. 10-2006-0075676. In the experiments as below, the cell culture densities of the monoclonal mesenchymal stem cells (MSCs) obtained by the subfractionation culturing method were varied in 50 cells/cm² (low density), 1,000 cells/cm² (medium density) and 4,000 cells/cm² (high density), respectively, thereby analyzing the characteristics of the cells.

### 1.1. Identification of Morphological Changes of MSC According to Cell Density

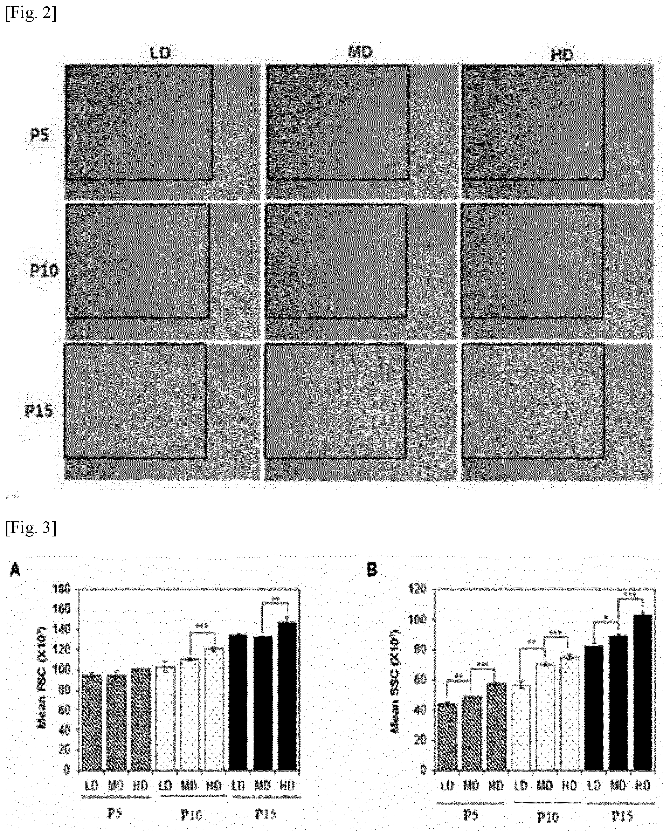

First, the experiments were conducted to identify the morphological changes of MSCs according to cell density in long-term culture. The MSCs having passage 5 (P5), passage 10 (P10) and passage 15 (P15) were used for the long-term culture conditions. The MSCs were inoculated in LG-DMEM under the condition of low density, medium density and high density, respectively. Thereafter, the morphological changes of the cells were observed through a microscope to determine whether or not the stem cells were aged. The results are illustrated in FIG. 2.

As illustrated in FIG. 2, the cell size and morphological pattern of P5 and P10 were different according to the cell density. In particular, P15 cultured under a high density culture condition had flat and enlarged MSCs. This morphology form indicates the typical aging of MSCs, which identifies that the cell density is controlled in the long-term culture, resulting in the control of MSC's aging.

### 1.2. Identification of MSC Size and Granularity According to Cell Density

In order to further identify the change of stem cells according to the cell density, the cell size and granularity, which were known to be increased in aged cells, were quantitatively analyzed by FACS. Thus, the results are illustrated in FIG. 3.

As illustrated in FIG. 3, the cell size did not show a significant difference at P5, but P10 and P15 showed significant differences according to the cell density. In particular, the cell sizes in P10 and P15 were significantly increased under a culture condition of the high cell density, thereby further promoting aging of cells. Similarly, the cell granularity also was significantly increased as the cell density was increased in all passages. Therefore, controlling cell density of MSC in long-term culture may be a factor to control aging of cells. Further, the cell culture density is lowered to improve the morphological changes in the late passage.

### 1.3. Identification of Aging of MSC According to Cultured Cell Density

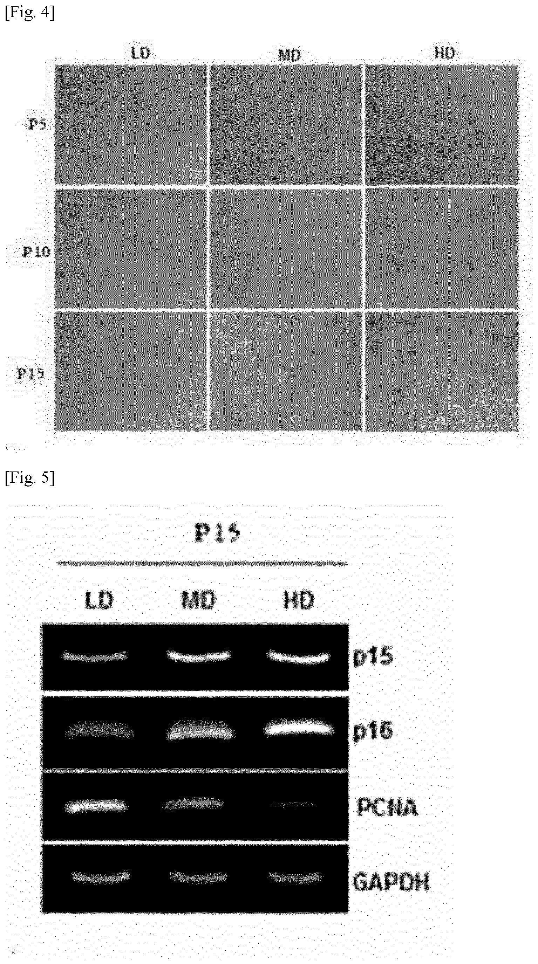

The beta-galactosidase-staining analysis was performed to identify whether the morphological changes confirmed in Examples 1.1 and 1.2 were actually an age-dependent phenomenon of MSC, which can selectively stain aging cells, and RT-PCR was performed to compare the expressions of aging-related genes P15, P16 and PCNA gene, a proliferation marker. The results are illustrated in FIGS. 4 and 5, respectively.

As illustrated in FIG. 4, P5 and P10 did not have the staining of aged cells at all cell densities, but P15 had the staining of aged cells markedly increased as the cell density increased. As illustrated in FIG. 5, in P15, gene expression of CDK inhibitors P15 and P16, which are genes related to aging, was increased as the culture density of cells increased, and PCNA, which is a proliferation marker, decreased.

These results demonstrate that the morphological changes of MSCs are related to the aging of MSCs and that the control of the cell culture density may control the aging of MSCs.

### 1.4. Identification of Change of Proliferation Ability of MSCs According to Culture Cell Density

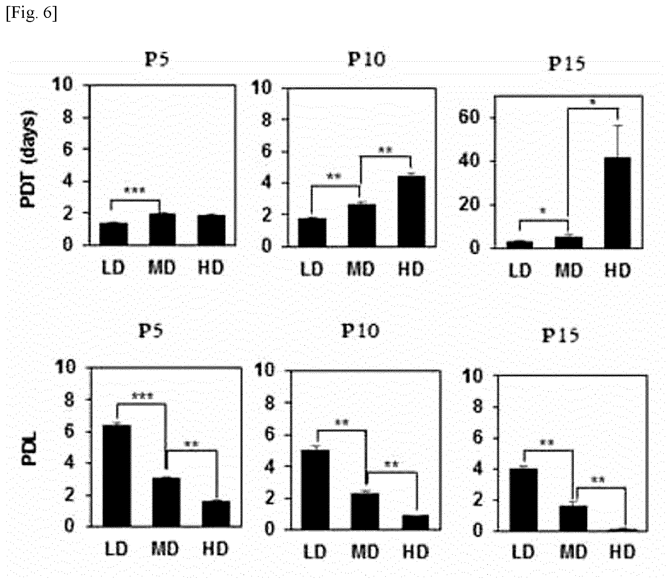

It is known that the proliferation ability of MSCs progressively decreases as cells are subcultured and aged. Therefore, the proliferation ability can be used as a criterion for identifying the aging of MSCs. Thus, the proliferation ability of MSCs according to the cell culture density was compared during long-term cell culture. The proliferation ability of each cell was determined by calculating the proliferation rate according to each passage using the number of cells which were initially inoculated and the number of cells which were obtained after the culture was completed. The results are shown in Table 1 and FIG. 6.

**[Table 1]**

| **Fold Increase** | | | |
|---|---|---|---|
| **(cell/cm²)** | **P5** | **P10** | **P15** |
| **50** | 88.4±6.5 | 34.3±5.0 | 16.4±1.3 |
| **1000** | 8.5±0.3 | 4.9±0.5 | 3.1±0.4 |
| **4000** | 3.0±0.1 | 1.9±0.1 | 1.1±0.1 |

As shown in Table 1, the fold increases were 88.4, 34.3 and 16.4 at P5, P10 and P15 in MSCs cultured at a low density. Meanwhile, the fold increases were 8.5, 4.9 and 3.1 in MSCs cultured at a medium density, and the fold increases were 3.0, 1.9, and 1.1 in MSCs cultured at a high density. As illustrated in FIG. 6, the PDT and the PDL also have the same pattern as the fold increase. These results indicate that the proliferation ability of MSCs may be maintained by lowering the cell density in long-term MSC culture and that even though performing the same subculture, the aging of MSCs may be inhibited and the lifespan of MSCs may be prolonged.

### 1.5. Identification of Change of Differentiation Potential of MSCs According to Culture Cell Density

The differentiation potentials according to P5 to P15 cultures were compared to identify whether culture cell density affects the ability of stem cells. The ability of stem cells to differentiate into adipocytes and osteocytes was identified. Qualitative and quantitative analyzes were performed at each passage and density. Specifically, NCS (Newborn Calf Serum) (Gibco), 10⁻⁷ mol dexamethasone (Sigma), 0.5 mM IBMX (Sigma), 10 µg/ml insulin (Sigma), and 100 µM indomethacin (Sigma) were added to a high glucose DMEM culture medium to prepare the adipocyte differentiation culture medium, and then the experiment was performed. After 7 days of differentiation, it was identified by Oil red O histochemical staining. After the Oil red O histochemical staining, it was eluted with isopropyl alcohol and measured at 500 nm and quantitatively analyzed.

The osteoclast differentiation culture medium was prepared and used by adding FBS (Gibco), 50 µg/ml ascorbic 2-phosphate (Sigma), 10⁻⁸ mol dexamethasone (Sigma) and 10 mM beta-glycerophosphate (Sigma) to α-MEM culture medium. After 21 days of differentiation, it was identified by Alizarin red S histochemical staining. After Alizarin red S histochemical staining, it was eluted with 10% acetic acid, measured at 405 nm and quantitatively analyzed. The adipocyte differentiation potential and the osteoclast differentiation potential were identified as described above. The results are illustrated in FIG. 7.

As illustrated in FIG. 7, the adipocyte differentiation potential decreased overall as the passage progressed, but the difference according to the density was not apparent. On the other hand, the osteoclast differentiation potential significantly decreased in the P15 culture group under the condition of high density. These results show that the osteoclast differentiation potential of MSCs may be maintained better when culturing at a low cell density.

### 1.6 Antigen Profile Analysis of MSCs According to Culture Cell Density

Experiments were performed to identify whether the cell culture density also affects the stem cell antigen expression. Flow cytometry was performed to identify the changes in positive and negative antigen expression according to each passage and culture density. The results are shown in Table 2.

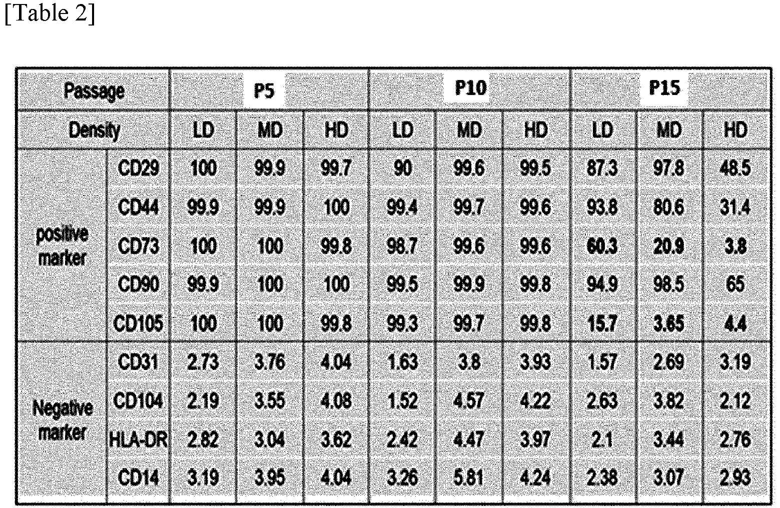

As shown in Table 2, the change of the expression of the negative marker was not clearly apparent, but the expression level of some positive markers was changed according to the cell culture density even in the same passage.

In particular, when cells were cultured at a high density in P15, the expression level of most positive markers was significantly decreased. Further, CD73 and CD105 showed negative expression. Thus, it was identified that cell culturing with a low cell density may be a critical factor.

### 1.7. Comparison of ROS Production and DNA Damage According to Culture Cell Density

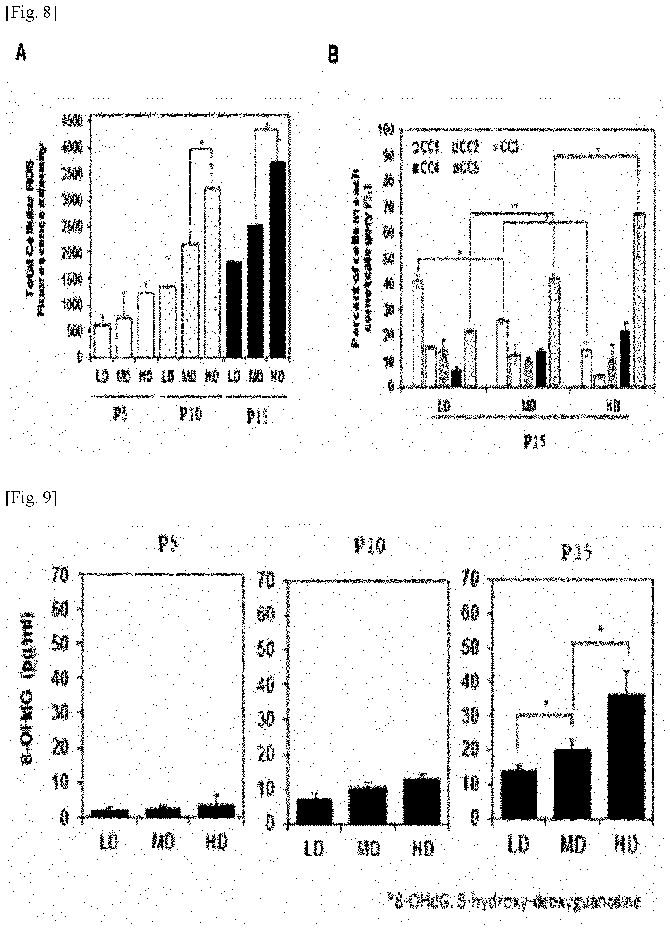

It is known that the decrease of mesenchymal stem cell function is associated with DNA damage. In particular, DNA damage induced by ROS, which is an active oxygen species, is known to promote aging of MSCs. Therefore, in order to identify whether total ROS production and DNA damage caused thereby are different according to culture density, fluorescence intensity analysis was performed to compare the total amount of cellular ROS according to passage and cell culture density. Comet analysis was performed to identify the degree of DNA damage. The results are illustrated in FIG. 8.

As illustrated in FIG. 8, total ROS production tended to increase as the cell culture density increased in all passages. In particular, ROS production significantly increased at P10 and P15 (A). In the comet analysis, the data were analyzed after classifying the data from CC1 with the weakest DNA damage to CC5 with the most severe DNA damage. The CC5 with the most severe DNA damage exhibited a significant increase as the cell culture density increased. On the other hand, the CC1 tended to decrease significantly as the cell density increased (B).

Further, in order to further identify whether ROS caused DNA damage, an experiment was conducted to identify the concentration of 8-OHdG which may identify DNA damage caused by ROS. The analysis method of 8-OHdG is as follows. 50 µl of the DNA sample collected from each cell was placed on an 8-OHdG conjugate coated plate and incubated at room temperature for 10 minutes. Then, an anti-8-OHdG antibody was added thereto and the mixture was incubated at room temperature for 1 hour. After washing three times, secondary antibody-enzyme conjugate was added to each well, and the mixture was incubated at room temperature for another 1 hour. After washing three times, a substrate solution was added thereto, and the mixture was incubated at room temperature for 30 minutes. Finally, a stop solution was added thereto. The absorbance intensity thereof was measured at 450 nm. The results are illustrated in FIG. 9.

As illustrated in FIG. 9, as the cell culture density increased, the concentration of 8-OHdG was significantly increased in P15 group in which DNA damage was most severe. These results demonstrate that ROS produced under the culture condition of the high density caused DNA damage to increase, thereby promoting aging of MSCs.

These results show that lowering the cell culture density may play a role in protecting MSCs from DNA damage which is caused by increased ROS production ofMSCs.

### 1.8. Identification of MSC Proliferation and ROS Production Ability According to Antioxidant Treatment

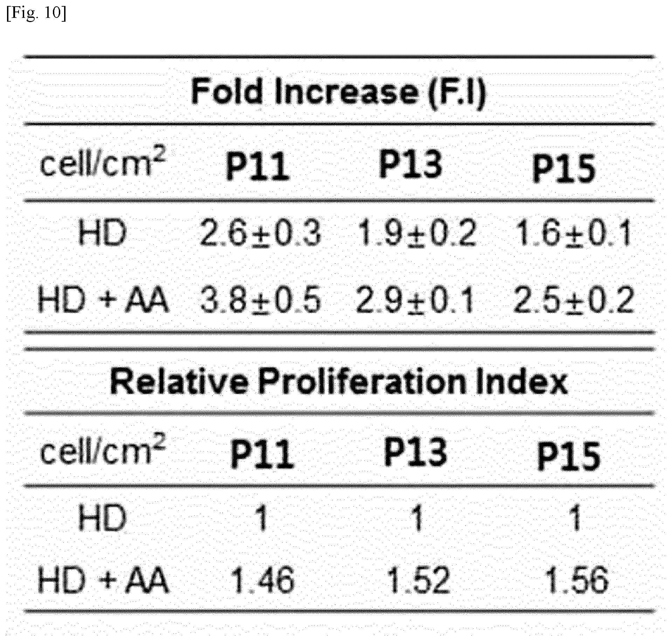

In order to identify whether ROS produced under the culture condition of high density affects the proliferation of MSCs, an experiment for eliminating ROS was performed. 25 µg/ml ascorbic acid, an antioxidant, was added to the medium under the culture condition of high density in P11 to P15. After the culture, the fold increase of proliferation rate was compared between the two groups. The results are illustrated in FIG. 10.

As illustrated in FIG. 10, the fold increase was 2.6, 1.9, and 1.6 at P11 to P15, respectively, in the high density culture condition. As passage number increased, the proliferation ability decreased, and as a result, aging began. However, treating with the antioxidant induced to maintain high proliferation ability at about 50% in all passages. In the antioxidant treatment group, the growth fold increase was 3.8, 2.9 and 2.5 in P11 to P15, respectively. The proliferation ability was maintained high even at P15.

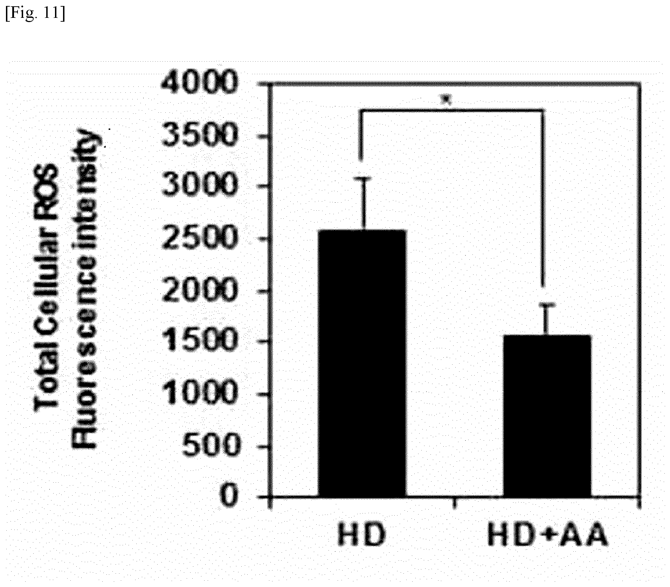

At the endpoint P15, ROS levels were confirmed between the high density culture condition alone and high density culture condition + antioxidant treatment groups. The results are illustrated in FIG. 11.

As illustrated in FIG. 11, the ROS level also decreased under the condition that proliferation was increased by treating ascorbic acid, an antioxidant. Therefore, MSC culture was preferably performed at a low cell density rather than a high density. ROS production induced by a high density of cell culture was eradicated with an antioxidant, thereby resulting in an increase of MSC proliferation ability. In other words, ROS at a high density inhibited MSC proliferation ability. As the cell density was lower, the ROS production was decreased, and the MSC proliferation ability was promoted.

To sum up, these results indicate that controlling the cell density at the density of 1,000 cells/cm² or less in culture conditions is important to maintain the proliferation, culture and stem cell ability of the monoclonal mesenchymal stem cells obtained through the subfractionation culturing method. Culture with an antioxidant inhibits oxidative stress induced by cell culture, thereby promoting MSC proliferation efficiently. In addition, stem cells of passage 15 or higher showed significant changes in cell morphology when compared to stem cells of passage 5 or 10, and results such as promoting aging of stem cells and decreasing differentiation potential were identified. It was identified that culture at a density of 1,000 cells/cm² or less with a low passage number was the most effective.

### <Example 2> Verification of Improved Subfractionation Culturing Method

Example 1 has identified that the control of cell density, passage control, and the addition of an antioxidant may be critical factors in the MSC culture obtained by the subfractionation culturing method. Therefore, the experiments were conducted to compare the proliferation ability of single colony MSCs and their effect of obtaining cells by varying the cell culture density of monoclonal mesenchymal stem cells obtained by the conventional method of the subfractionation culturing method described in Korean Patent Application No. 10-2006-0075676, and using a medium containing ascorbic acid as an antioxidant.

Korean Patent Application No. 10-2006-0075676 as previously disclosed, discloses that colonies, a single cell group, were transferred at 100 to 600 cells per well, and then were cultured at 4,000 cells per cm² in their subculture.

In addition, Korean Patent Application No. 10-2007-0053298, which discloses a therapeutic agent for graft-versus-host disease using mesenchymal bone marrow cells obtained through the subfractionation culturing method, merely discloses that a single cell group obtained by the subfractionation culturing method is transferred to a 6-well culture vessel corresponding to passage 1 at 100 to 600 cells, but does not disclose the configuration and effects of repetitive culture density control of individual cells other than colonies after passage 2. According to the conventional subfractionation culturing method described in the above application, culture of at least passage 10 needs to be performed to obtain a sufficient amount of monoclonal stem cells with effects of preventing, treating, or alleviating graft-versus-host disease. On the other hand, in the improved subfractionation culturing method of the present disclosure, it is possible to effectively obtain a large amount of the desired monoclonal stem cells through low cell density conditions after passage 2 and low subcultures such as passages 2 to 13.

Specifically, in this improvement method, colonies of passage 1 (P1) obtained through the subfractionation culturing method were cultured. Thereafter, in the subculture after passage 2 (P2), cells were seeded in 1,000 cells/cm² or less which is a low density. These were compared with the effect of 4,000 cells/cm² cells culture. Further, α-MEM containing antioxidant and LG-DMEM containing no antioxidant were used as a cell culture medium, thereby comparing their effects of cell proliferation.

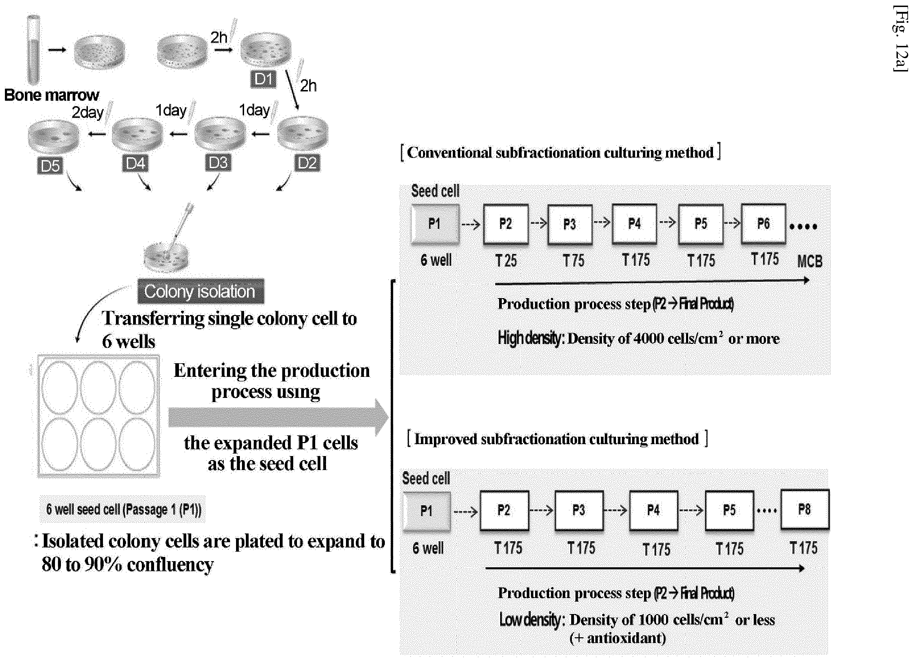

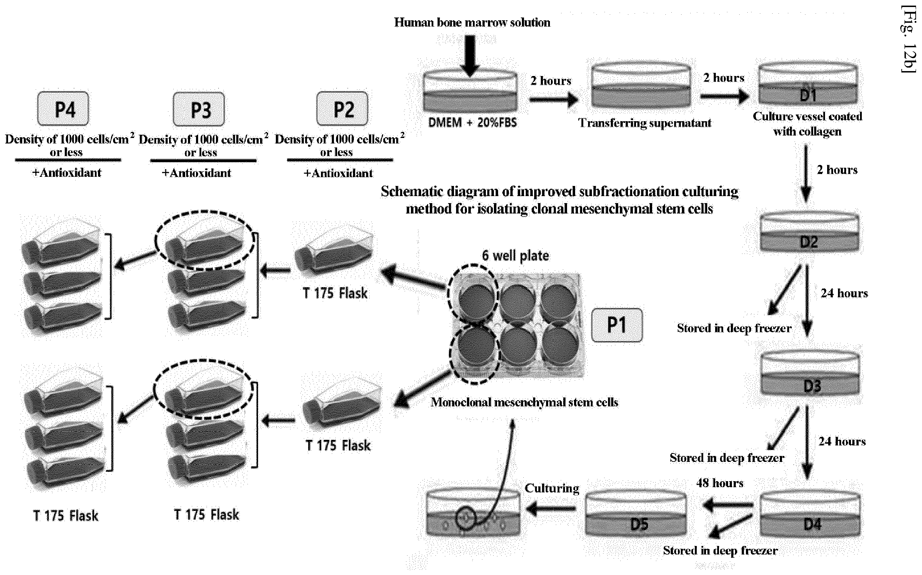

Experimental groups for identifying the effect of the improved subfractionation culturing method are shown in Table 3 below. The improved processes are schematically illustrated in FIGS. 12A and 12B.

As illustrated in FIG. 12A, until the process of obtaining passage 1, the conventional subfractionation culturing method and the improved subfractionation culturing method proceeded in the same process. However, the improved subfractionation culturing method used the expanded passage 1 cell as a seed cell. Thus, the subculture process after the culture is different from the conventional subfractionation culturing method. The conventional subfractionation culturing method performs high-density subculture of 4,000 cells/cm² or more for the purpose of obtaining lots of cells without awareness of the density condition. However, the improved subfractionation culturing method is capable of obtaining a sufficient amount of the final products only with the culture after passage 2 and passage 9 or less by controlling the density of subculture to a low density of 1,000 cells/cm² or less. Expansion culture may be performed up to passage 13 in order to obtain more monoclonal stem cells. The culture process after passage 2 is shown in detail in FIG. 12B.

**[Table 3]**

| **Group** | Culture density | Medium condition | **Passage** | Subculturing period **(day)** | Medium exchange |
|---|---|---|---|---|---|
| 4000LG | 4000cells/cm² | LG-DMEM | **P2-P5** | 3-4 | X |
| 4000alpha | 4000cells/cm² | alpha-MEM | | 3-4 | X |
| 1000LG | 1000cells/cm² | LG-DMEM | | 7 | O(every 3-4days) |
| 1000alpha | 1000cells/cm² | alpha-MEM | | 7 | O(every 3-4days) |

Cell lines were separated by the subfractionation culturing method, which were named as SCM01 to SCM08, respectively.

### 2.1. Identification of Proliferation Effect According to Cell Line Density and Medium

The cells were cultured using the SCM01 to SCM08 cell lines. To identify the cell proliferation effect according to subculture, the cell number, population doubling time (PDT) and population doubling level (PDL) were compared, respectively. The results are illustrated in FIGS. 13 to 20.

As illustrated in FIGS. 13 to 20, the cell proliferation effect of all experimental groups inoculated and cultured with a cell density of 1,000 cells per cm² was superior to those of experimental groups inoculated and cultured with a cell density of 4,000 cells per cm². Furthermore, even in the same 1,000 cell density group, the 1,000-alpha experimental group cultured in α-MEM containing ascorbic acid as an antioxidant showed more significant cell proliferation effect than other groups.

### 2.2 Comparison of Proliferation Effect According to Cell Line Density

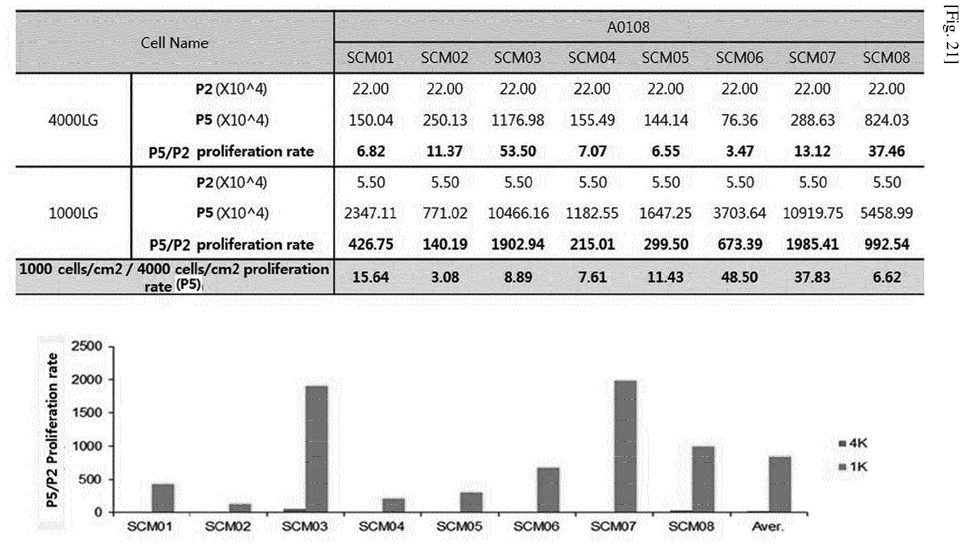

For a more accurate comparison of the proliferation rate according to the number of cultured cells, LG-DMEM and α-MEM, respectively, were set as a culture medium, and the cell proliferation effect according to the cell inoculation density of 1,000 or 4,000 cells per cm² was compared. The results are illustrated in FIGS. 21 to 24.

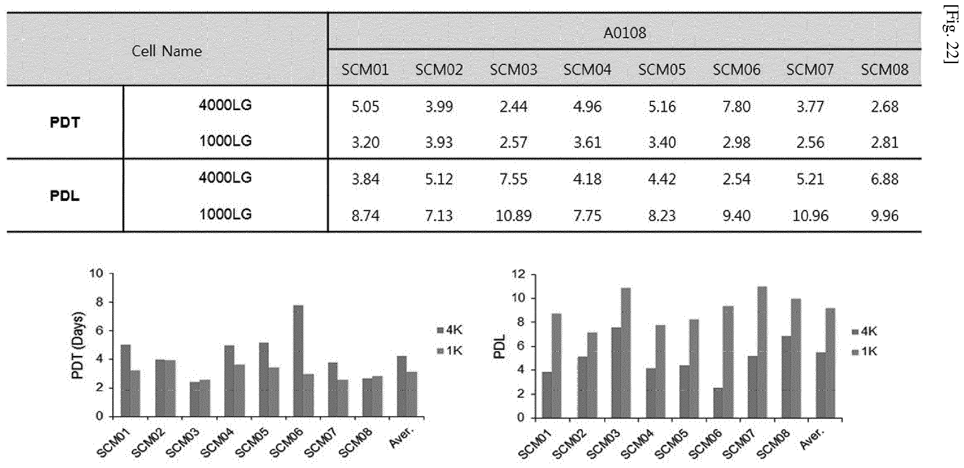

As illustrated in FIG. 21, when the SCM01 to SCM08 cell lines inoculated with 1,000 cells/cm² were cultured in LG-DMEM, the proliferation rate of P2 to P5 was significantly higher than that of the group inoculated with 4,000 cells/cm². The proliferation rate of the group inoculated with 1,000 cells/cm² was at least 3.08 to at most 48.50 times compared with those of the group inoculated with 4,000 cells/cm² in passage 5 (P5). Further, as illustrated in FIG. 22, the PDT value of 1,000 cells/cm² cell inoculation group was also lower or similar to those of 4,000 cells/cm² cell line inoculation in all cell lines, and the PDL value was higher than those of 4,000 cells/cm² cell inoculation in all cell lines.

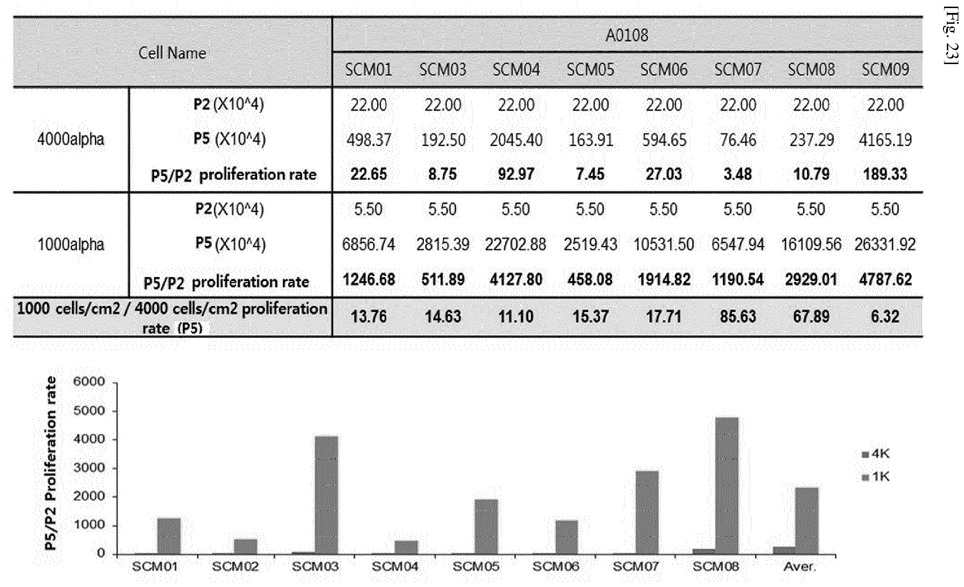

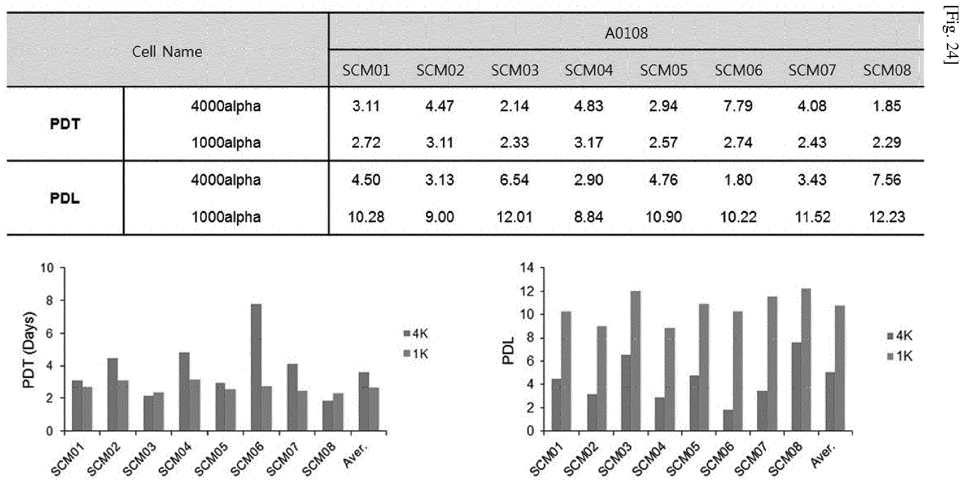

Further, as illustrated in FIG. 23, when all SCM01 to SCM08 cell lines cultured in α-MEM were inoculated and cultured with 1,000 cells/cm², their tendency was similar to those of DMEM experimental groups. The proliferation rate of the group inoculated with 1,000 cells/cm² was at least 6.32 to at most 85.63 times compared with those of the group inoculated with 4,000 cells/cm² in passage 5 (P5). Further, as illustrated in FIG. 24, the PDT value of 1,000 cells/cm² cell inoculation group was also lower or similar to those of 4,000 cells/cm² inoculation in all cell lines, and the PDL value was higher than those of 4000 cells/cm² cell inoculation in all cell lines.

These results demonstrate that the inoculation with 1,000 cells/cm² or less may induce rapid proliferation of monoclonal mesenchymal stem cells compared to high density cell inoculation culture of 4,000 cells per cm².

### 2.3 Comparison of Proliferation Effect According to Culture Medium

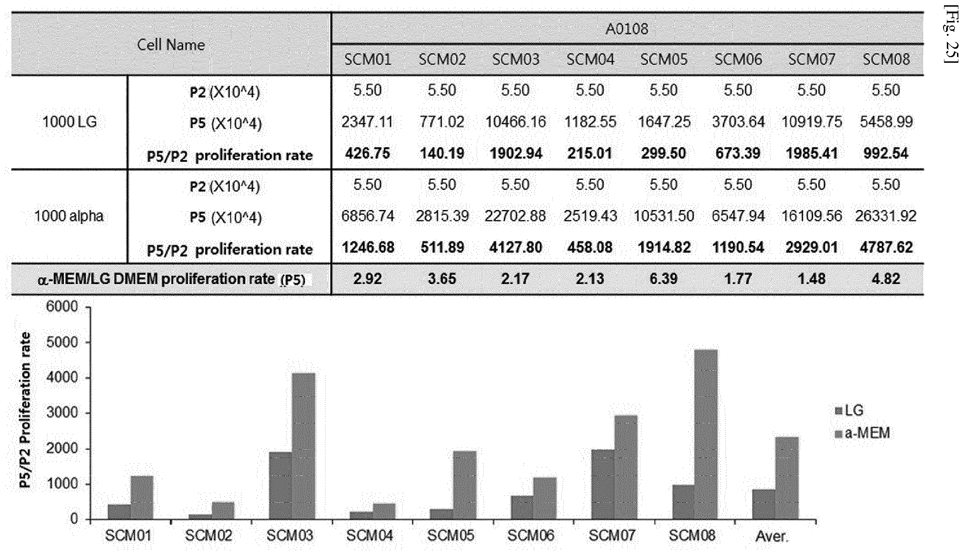

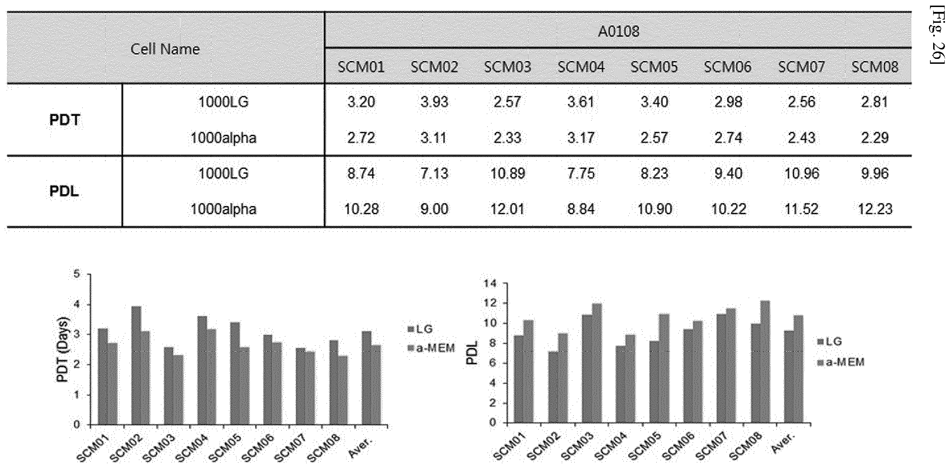

Example 2.2 identified that 1,000 cells/cm² culture showed excellent proliferation effect compared with 4,000 cells/cm² culture. Therefore, the experiment was conducted to compare the cell proliferation effect while the number of cells was set as 1,000 cells/cm², and the medium varied as a variable. Thus, the proliferation effect was further verified according to the culture medium conditions. The results are illustrated in FIGS. 25 and 26.

As illustrated in FIG. 25, the cell proliferation rates were compared between α-MEM and DMEM. The results showed that the cell proliferation rate of the experimental group using α-MEM was at least 1.77 to 6.39 times compared with those of the experimental group using LG-DMEM. Further, as illustrated in FIG. 26, the PDT was low in all α-MEM experimental groups and the PDL was increased in all α-MEM experimental groups.

These results indicate that the cell proliferation efficiency may be maximized by culturing cells using a medium containing an antioxidant in addition to control of the cell inoculation density of 1,000 cells or less per cm² and culture of the cells in low passages.

### <Example 3> Establishment of Improvement Process

Examples, as described above, identified that the control of cell density and the addition of an antioxidant might be important factors in the MSC culture. In addition to the conventional process of the subfractionation culturing method described in Korean Patent Application Nos. 10-2006-0075676 and 10-2007-0053298, the improved process was established by varying the cell culture density and the medium conditions for effectively obtaining single colony mesenchymal stem cells at low passages. These are collectively shown in the following Table 4 (culture conditions using DMEM) and Table 5 (culture conditions using α-MEM).

**[Table 4]**

| **Process** | **Items** | **Fresh Product** | **Frozen Product** |
|---|---|---|---|
| Bone marrow ∼MCB * seed cell (except P.1) Subculturing | Culture medium | DMEM | DMEM |
| | Antibiotic (concentration) | Penicillin-streptomycin | Penicillin-streptomycin |
| | | Penicillin (100units/mL) | Penicillin (100units/mL) |
| | | Streptomycin (100 µg/mL) | Streptomycin (100 µg/mL) |
| | Cell culture density | 50∼1000cells/cm^{a} | 50∼1000cells/cm^{a} |
| | period | 3 ∼ 14 days | 3 ∼ 14 days |
| | Passage | P1 ∼ P13 | P1 ∼ P13 |
| **MCB∼WCB** | Culture medium | DMEM | DMEM |
| | Antibiotic (concentration) | Penicillin-streptomycin | Penicillin-streptomycin |
| | | Penicillin (100units/mL) | Penicillin (100units/mL) |
| | | Streptomycin (100*µ*g/mL) | Streptomycin (100 *µg*/ml) |
| | Cell culture density | 50∼1000cells/cm² | 50∼1000cells/cm² |
| | Subculturing period | 3 ∼ 14 days | 3 ∼ 14 days |
| | Passage | P3∼P9 | P3∼P9 |
| Final product | Culture medium | DMEM | DMEM |
| | Antibiotic (concentration) | Penicillin-streptomycin | Penicillin-streptomycin |
| | | Penicillin (100units/mL) | Penicillin (100units/mL) |
| | | Streptomycin (100 *µ*g/mL) | Streptomycin (100 *µ*g/mL) |
| | Cell culture density | 50∼1000cells/cm² | 50∼1000cells/cm² |
| | Subculturing period | 3 ∼ 14 days | 3 ∼ 14 days |
| | Passage | P6∼P13 | P6∼P13 |

**[Table 5]**

| **Process** | **Items** | **Fresh Product** | **Frozen Product** |
|---|---|---|---|
| **Bone marrow ∼MCB *seed cell (except P.1)** | **Culture medium** | **α-MEM** | **α-MEM** |
| | **Antibiotic (concentration)** | **Gentamicin** (20 *µ*g/mL) | **Gentamicin** (20 *µ*g/mL) |
| | Cell culture density | 50∼1000cells/cm² | 50∼1000cells/cm² |
| | Subculturing period | 3 ∼ 14 days | 3 ∼ 14 days |
| | Passage | P1∼ P13 | P1∼ P13 |
| **MCB∼WCB** | Culture medium | **α-MEM** | **α-MEM** |
| | Antibiotic (concentration) | Gentamicin (20 *µ*g/mL) | Gentamicin (20 *µ*g/mL) |
| | Cell culture density | 50∼1000cells/cm³ | 50∼1000cells/cm² |
| | Subculturing period | 3 - 14 days | 3 ∼ 14 days |
| | Passage | P3∼P9 | P3-P9 |
| Final product | Culture medium | **α-MEM** | α-MEM |
| | Antibiotic (concentration) | Gentamicin (20 *µ*g/mL) | Gentamicin (20 µg/mL) |
| | Cell culture density | 50∼1000cells/cm² | 50∼1000cells/cm² |
| | Subculturing period | 3 ∼ 14 days | 3 ∼ 14 days |
| | Passage | P6-P13 | P6∼P13 |

More specifically, the subfractionation culture process and proliferation culture of the bone marrow-derived mesenchymal stem cells of the present disclosure were performed as follows.

The hip of a bone marrow donor was anesthetized with local anesthetics. Then, the bone marrow was collected by piercing the needle into the hip bone. 14 ml of Dulbecco's modified Eagle's medium (DMEM, GIBCO-BRL, Life-technologies, MD, USA) containing 20% FBS and 1% penicillin/streptomycin, and 1 ml of human bone marrow were placed in a 100 mm culture vessel, and the mixture was cultured in a CO₂ cell incubator at 37°C for 2 hours. After the culture, the culture vessel was slightly tilted to one side, and only the supernatant of the culture vessel was transferred to a new vessel while the cells attached to the bottom were prevented from falling down.

The same procedure was repeated one more time, and the resulting culture solution was transferred to a culture vessel (Becton Dickinson) coated with collagen on the bottom thereof and cultured at 37°C for 2 hours. The culture solution was transferred to a new vessel coated with collagen. After 24 hours, the culture solution was transferred to a new vessel. Again, after 24 hours, the culture solution was transferred to a new vessel. Finally, after 48 hours, the culture solution was transferred to a new vessel. Then, it was visually identified that the remaining cells were grown and adhered to the bottom of the culture vessel. It can be assumed that cells which can come up to this step through the previous several subfractionation steps have a much smaller specific gravity of cells than other cells.

After about 10 days to about 14 days, the cells formed a single colony. These monoclonal cell groups were treated with trypsin to be isolated. Then, the cells were transferred to a 6-well culture vessel. The cells were cultured in a 5% CO₂ cell incubator at 37°C for 4 to 5 days. Then, when the cells were grown to about 80%, the cells were treated with 0.05% trypsin/1 mM EDTA (GIBCO-BRL), thereby obtaining the cells. Then, the cells were transferred to a T175 culture vessel and subcultured at a low cell density.

When the cells were cultured at a cell density which was lowered to 1,000 cells/cm² in the early passage but all the other processes were regulated in the same manner, the proliferation ability and stem cell characteristics of MSCs were excellently maintained to induce efficient proliferation even in the same passage. In particular, when the cells were cultured at a lowered cell density, it may exclude a process of producing a WCB (Working Cell Bank) in MSCs, which is required in the conventional process, thereby shortening the cell production period efficiently. In particular, when the passage is reduced, cells with relatively less aging may be obtained in a large amount. It is expected that such cells are used as a therapeutic agent to lead to excellent therapeutic efficacy.

Further, when α-MEM supplemented with an antioxidant is used as a culture medium, the antioxidant treatment may effectively reduce the ROS stress induced in high density cell culture and restore the cell proliferation ability of MSCs, thereby shortening the cell passage significantly and rapidly and stably obtaining single colony MSCs in a fresh state without aging, which maintains the characteristics of MSCs.

In summary, the low density cell culture may not only obtain a large number of cells in the short term, thereby simplifying the production process, but also obtain cells with non-aging to maintain the intact characteristics of MSCs in the long-term culture. Therefore, this leads to high quality stem cell production.

Accordingly, stem cells obtained through the improved method constructed through the above examples were used in the experiment for the treatment of graft-versus-host disease below.

### <Example 4> Identification of Therapeutic Effects of Graft-versus-host disease of Stem Cells obtained through Improved Subfractionation Culturing Method

### 4.1 Preparation of Graft-versus-host disease Animal Models

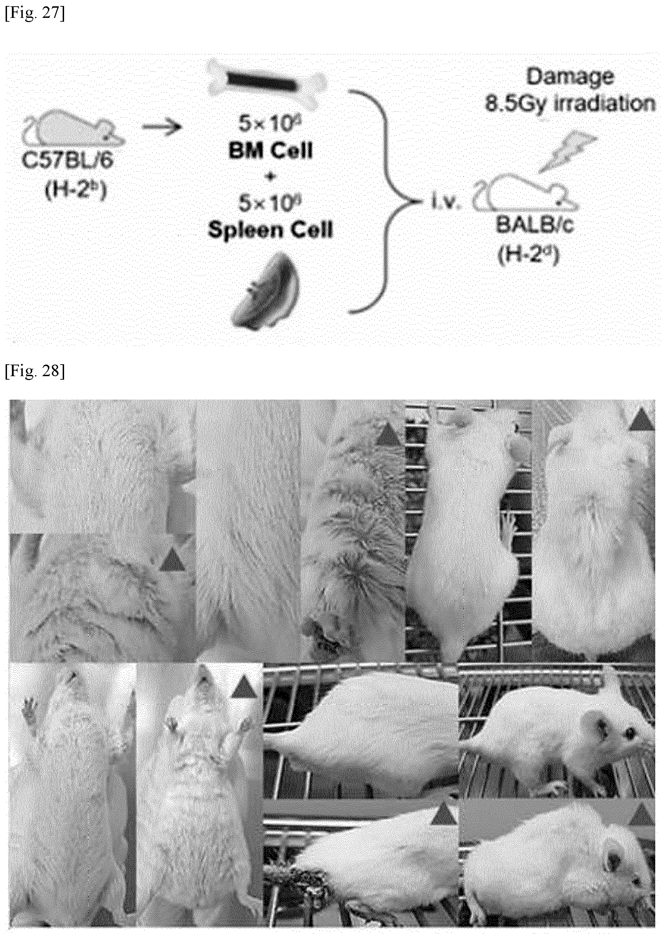

FIG. 27 shows a method of preparing an animal model of graft-versus-host disease. First, BALB/c mice were irradiated with 8.5 Gy of radioactivity. After 24 hours, bone marrow cells (5 × 10⁶/0.1 ml) and splenocytes (5 × 10⁶/0.1 ml) were extracted from female C57BL/6N, and were injected through the vein of the irradiated BALB/c mice. In order to identify whether the graft-versus-host disease mouse model was established, the skin, fur texture, ascites, diarrhea, and hunched back were observed in the prepared mice, and the clinical symptoms of the graft-versus-host disease were identified. The prepared mice are shown in FIG. 28.

As shown in FIG. 28, the mice prepared according to the preparation method of the present disclosure exhibited a phenomenon that the hair was rough, split and fallen out compared to the control group, and it was identified that the hair was yellowish. In addition, the abdomen was swelled and ascites accumulates, bloody stool symptoms appeared, and hunched back was observed. In addition, it was identified that the amount of exercise and food also decreased compared to the control group.

In addition, flow cytometric analysis and immunohistochemical analysis were performed to verify the establishment of an animal model for graft-versus-host disease.

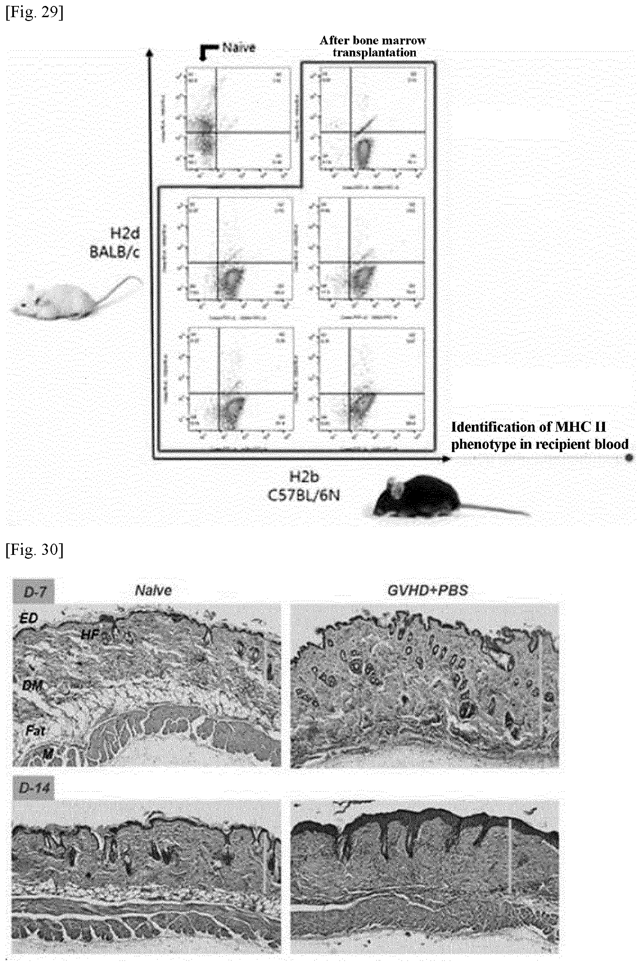

First, the phenotype, immune cells, and inflammatory cells of mice were observed using a flow cytometer, and the results are shown in FIG. 29.

As shown in FIG. 29, the graft-versus-host disease mice prepared by the preparation method of the present disclosure exhibited the MHC II phenotype in the blood of C57BL/6 recipients compared to Naive mice.

Immunohistochemical analysis through tissue staining was performed on the 7th and 14th days after induction of graft-versus-host disease, and the results are shown in FIG. 30.

As shown in FIG. 30, it was identified that the graft-versus-host disease mice prepared by the preparation method of the present disclosure had a thicker dermal layer compared to the Naive mice. In addition, monocyte infiltration was observed in dyskeratotic cells and the periphery of blood vessels. In particular, on the 14th day after induction of graft-versus-host disease, it was identified that both the epidermal and dermal layers were developed thicker than those of the Naive mice, the fat layer under the dermal layer collapsed, and there was damage to the hair follicles or sebaceous glands.

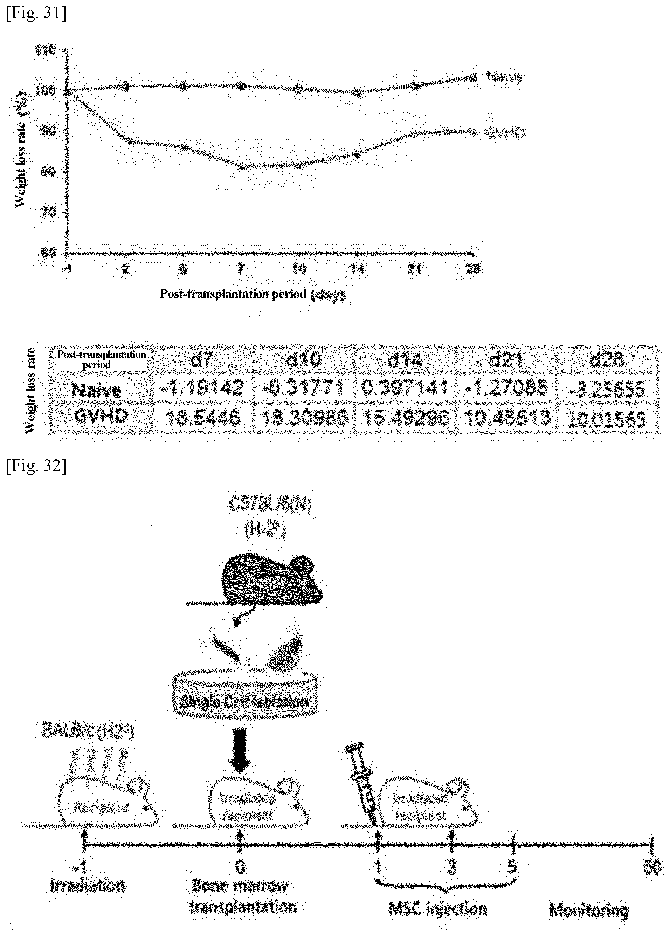

In order to identify whether the graft-versus-host disease-induced mice had a change in weight compared to the Naive mice, the change in weight was measured for 4 weeks after induction of the disease. The results are shown in FIG. 31.

As shown in FIG. 31, it was identified that the graft-versus-host disease-induced mice lost weight compared to the Naive mice, and showed the greatest change in weight on the 10th day after transplantation.

The above results indicate that the graft-versus-host disease mouse establishment was effectively achieved.

The preparation of the graft-versus-host disease animal model of the present disclosure and the subsequent treatment method are schematically and briefly shown in FIG. 32.

### 4.2. Identification of Therapeutic effects on Graft-versus-host disease compared to GCM (gradient centrifugation method)

### 4.2.1. Stem Cell Production and Administration by Improved Subfractionation Culturing Method

According to the improved subfractionation culturing method of Example 3, the cell culture density was set as 1,000 cells/cm² or less, and the monoclonal mesenchymal stem cells were obtained using α-MEM containing an antioxidant. Gentamicin was added as an antibiotic, and was used as α-MEM culture medium *(see* Table 5). Hereinafter, the stem cells obtained through the improved subfractionation culturing method of low density of 1,000 cells/cm² or less and antioxidant conditions were named as "cMSC1." As a control group, a group to which stem cells isolated by the GCM were administered to the graft-versus-host disease-induced mice was set, named as "GCM-MSC," and administered in the same manner as cMSC1.

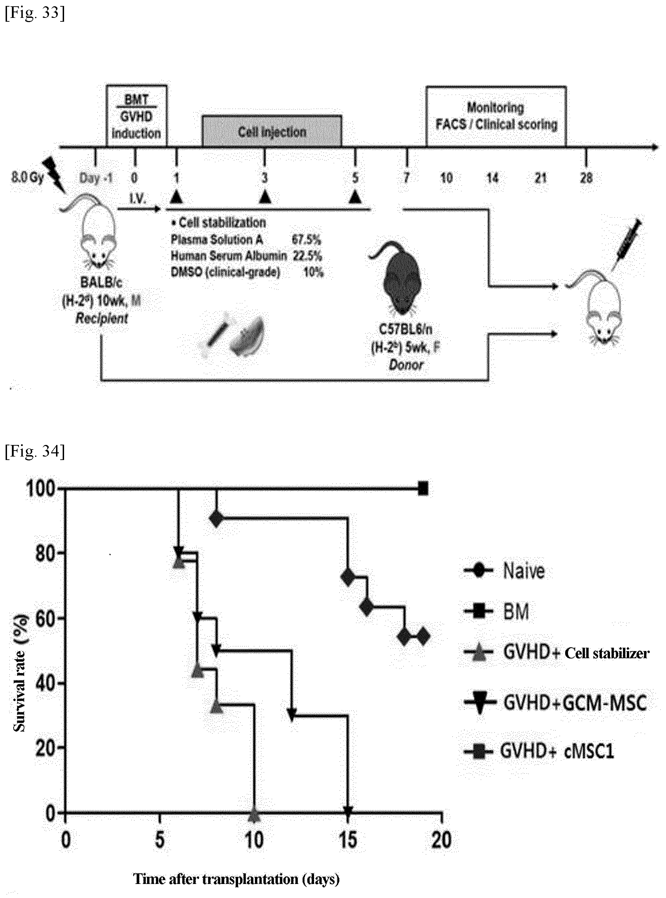

In order to identify the therapeutic effect on the graft-versus-host disease according to the cMSC1 injection of the present disclosure, 1 × 10⁶ cMSC1 was administered to the BALB/c mice of Example 4.1 in which the graft-versus-host disease was induced on the 1st, 3rd, and 5th days, and the therapeutic efficacy was observed. Its specific treatment protocol is shown in FIG. 33. The experimental groups G1 to G5 used for the treatment of graft-versus-host disease and the number of subjects belonging to each experimental group are shown in Table 6 below, and the doses administered on the 1st, 3rd, and 5th days (administration on the 1st day = 1^{st}, administration on the 3rd day = 2^{nd}, and administration on the 5th day = 3^{rd}) are shown in Table 7.

**[Table 6]**

| | *Group* | *head* |
|---|---|---|
| G1 | Naive | 5 |
| G2 | BM(Bone Marrow) | 5 |
| G3 | GVHD + Cell stabilizer | 11 |
| G4 | GVHD + GCM-MSC | 11 |
| G5 | GVHD + SCM-cMSC | 11 |

**[Table 7]**

| | 1st | *2nd* | 3rd | *Total* |
|---|---|---|---|---|
| Dose | 200µℓ | 200µℓ | 200µℓ | 200µℓ |
| Number of cells | 3.3 × 10⁵/200µℓ | 3.3 × 10⁵/200µℓ | 3.3 × 10⁵/200µℓ | 1 × 10⁶/200µℓ |

### 4.2.2. Identification of Therapeutic Effects by Administration of cMSC1

### Measurement of Survival Rate of Graft-versus-host disease Mice

After induction of the graft-versus-host disease, the survival rates of G1 to G5 including the control group and the experimental group were identified for about 20 days. The results are shown in Table 8 and FIG. 34.

**[Table 8]**

| Time after induction (days) | | 6 | 7 | 10 | 15 |
|---|---|---|---|---|---|
| Survival rate (%) | Naïve (G1) | 100 | 100 | 100 | 100 |
| | BM(G2) | 100 | 100 | 100 | 100 |
| | GVHD + Cell **stabilizer(G3)** | 80 | 40 | 0 | 0 |
| | GVHD+GCM-MSC (G4) | 80 | 60 | 50 | 0 |
| | GVHD+cMSC1 (G5) | 100 | 100 | 90 | 70 |

As shown in FIG. 34 and Table 8, in the G3 experimental group administered only the cell stabilizer to the graft-versus-host disease mice, all the mice died on the 10th day, whereas in the G4 experimental group administered GCM-MSC, 50% of mice survived on the 10th day, all the mice died on the 15th day. In the G5 experimental group administered with cMSC1, 90% and 70% survived on the 10th and 15th days, respectively. When clonal stem cells cultured at a low concentration in α-MEM, an improved subfractionation culture, were applied to the corresponding disease, it was identified that the survival rate was effectively improved.

### Identification of Clinical Symptom Improvement Effect of Graft-versus-host disease Mice

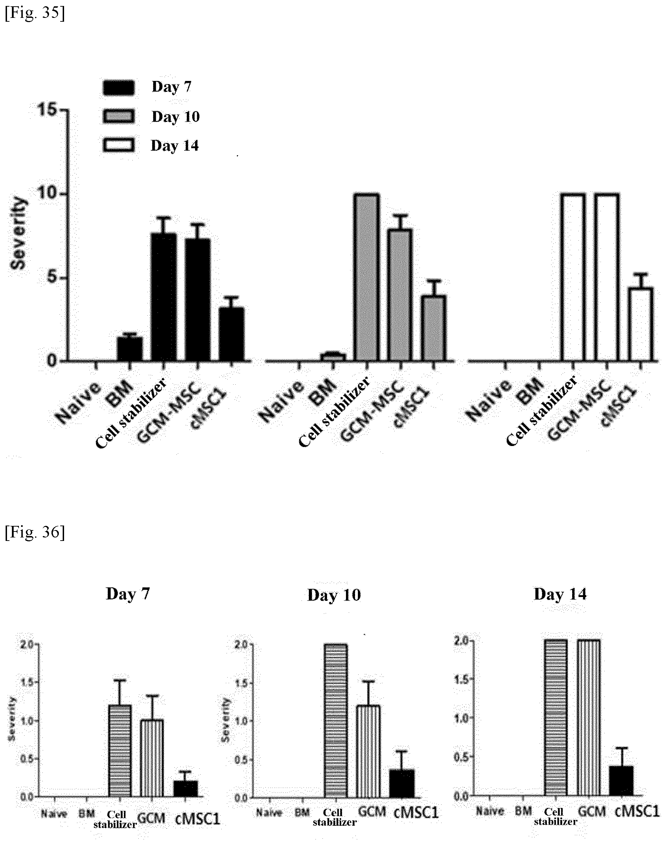

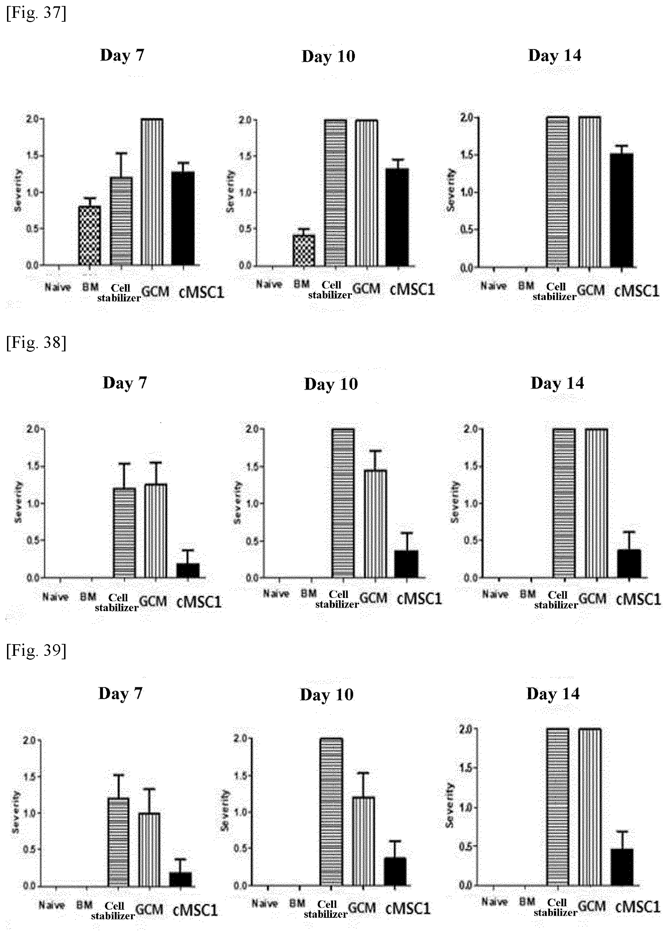

The effect of improving the clinical symptoms of graft-versus-host disease according to the administration of cMSC1 of the present disclosure was identified. According to the criteria in Table 9 below, the clinical symptoms of the experimental animals were numerically calculated as 0 to 2 points for each item and calculated as a total of 10 points. The results are shown in FIGS. 35 to 40.

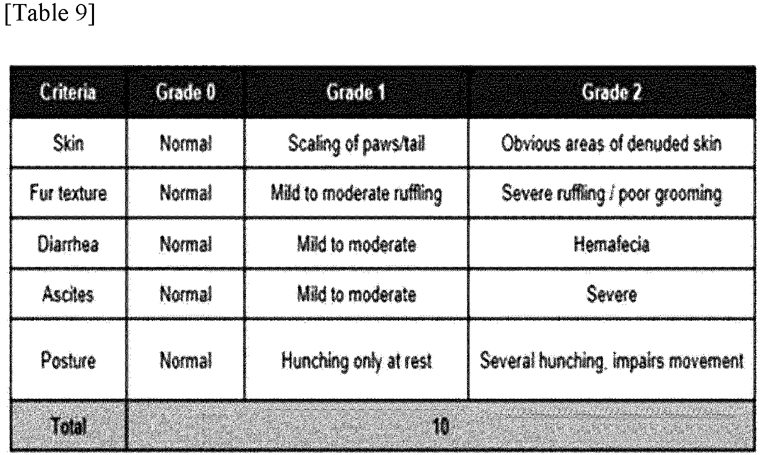

As shown in FIG. 35, it was identified that the mice administered with cMSC1 of the present disclosure had the lowest clinical score evaluated on days 7, 10, and 14 after induction of graft-versus-host disease.

FIGS. 36 to 40 are views showing scores for clinical symptoms for each item, each of which shows results for skin, fur texture, diarrhea, ascites, and posture (hunched back) of mice. As can be identified in FIGS. 36 to 40, in all clinical symptoms, the mice administered with cMSC1 of the present disclosure showed a remarkable effect of improving clinical symptoms compared to the experimental group administered only with the cell stabilizer. In addition, it showed a remarkably excellent effect when compared to the group administered with GCM-MSC.

Through the above results, it was identified that the clonal stem cells obtained by a culturing method performed at a low concentration in α-MEM, an improved subfractionation culturing method, have the effect of increasing the survival rate of graft-versus-host disease mice and improving various clinical symptoms, and thus may be beneficially used as a graft-versus-host disease therapeutic agent.

### <Example 5> Comparison of Therapeutic Effect of Stem Cells obtained through Improved Subfractionation Culturing Method

### 5.1. Comparison of cell characteristics of stem cells obtained through improved subfractionation culturing method and stem cells obtained through conventional subfractionation culturing method

cMSC1 is a mesenchymal stem cell obtained through an improved subfractionation culturing method having a low density and antioxidant medium conditions. As identified above, cMSC1 has superiority in that stem cell characteristics and proliferation ability are effectively maintained compared to mesenchymal stem cells obtained by the conventional subfractionation culturing method. A comparative experiment was conducted to identify whether cMSC1 has superiority as a therapeutic agent for graft-versus-host disease compared to the stem cells isolated by the conventional subfractionation culturing method.

As comparative groups, the stem cells (hereinafter referred to as "cMSC2") in which antioxidants were not added and which separated by the conventional subfractionation culturing method with a cultured cell number of 4,000 cells/cm² or more per area, and the monoclonal mesenchymal stem cells (cMSC1) obtained by culturing the cMSC1 at a low density in α-MEM supplemented with an antioxidant based on the same subfractionation method as the stem cell were used, which are summarized in Table 10.

**[Table 10]**

| Cell name | Media used | Number of cells cultured per area | Culture period | Particulars |
|---|---|---|---|---|
| cMSC2 | DMEM | 4000cells/cm² | 3-4 days | 4000LG |
| cMSC1 | α-MEM | 1000 cells /cm² | 7 days | Addition of antioxidant to medium 1000a |

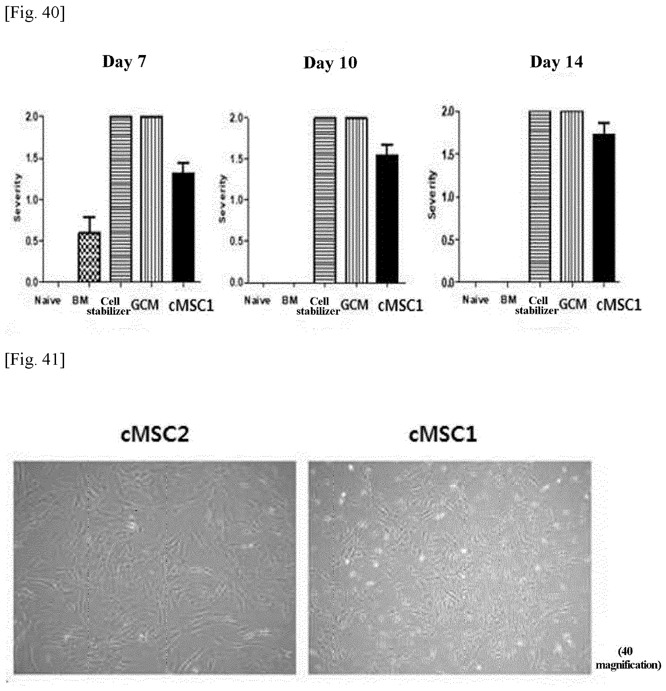

The number of cells and cell size of cMSC2 and cMSC1 of the present disclosure were identified through a microscope. The results are shown in FIG. 41. As shown in FIG. 41, it was identified that the size and number of cells were different between cMSC2 and cMSC 1 of the present disclosure obtained by different production processes.

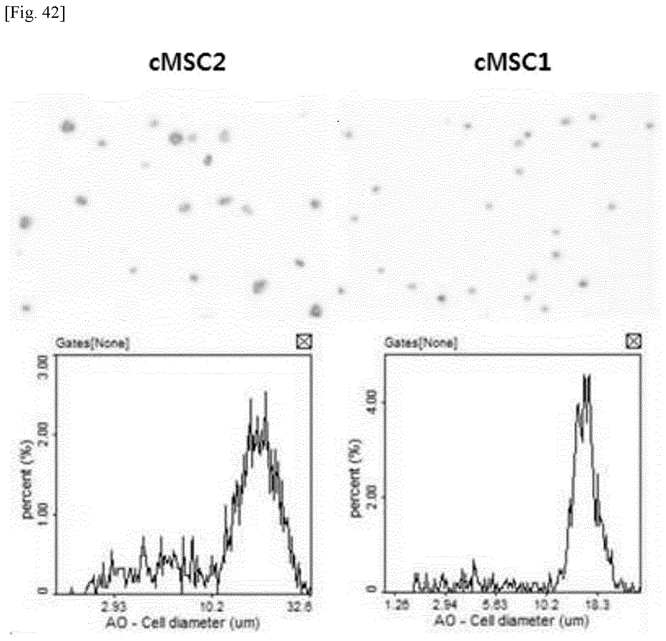

In order to verify the size difference between the two cells, the cell sizes were identified using a Nucleo Counter NC-250 device. More specifically, because mesenchymal stem cells are characterized by adherent growth to plastic plates, trypsin-EDTA was used to dissociate cells from plastic plates. Pictures of the cells were taken to identify whether there is any difference in the state of the cells when the cells were circular. The cell sizes were identified using a Nucleo Counter NC-250 device. The results are shown in FIG. 42 and Table 11.

**Table 11**

| Cell Name | cMSC2 | cMSC1 |
|---|---|---|
| Estimated Cell Diameter (µm) | 19.5 | 15.9 |
| Cell Diameter Standard Deviation (µm) | 14.8 | 10.2 |

As shown in FIG. 42, it was possible to identify the size difference between the two cells even in the circular state. When the cell diameter was identified through the device, cMSC2 was found to have various cell sizes ranging from 10.2 to 32.6 µm. On the other hand, cMSC1 was found to have cell sizes ranging from 10.2 to 18.3 µm, which was smaller and homogeneous in cell size than cMSC2.

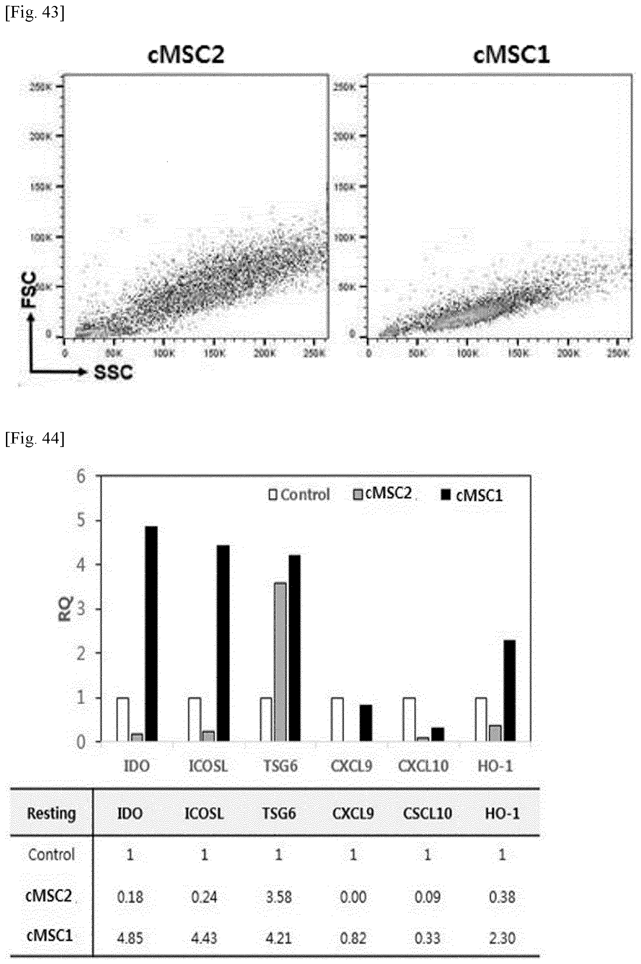

In addition, when the forward-scattered/side-scattered light (FSC/SSC) values were identically designated using a flow cytometer (FACS), it was identified whether the sizes of the cells were different also in flow cytometry. The results are shown in FIG. 43.

As shown in FIG. 43, it was identified that cMSC2 has a large cell size and thus cells are generally widely distributed, whereas cMSC1 has a small cell size and thus cells are distributed within 200 K of side-scattered light (SSC).

As described above, as the cell size of cMSC1 was smaller and homogeneously formed, the amount of cytokines secreted while mesenchymal stem cells were cultured was changed. Thus, it was expected that the therapeutic effect on graft-versus-host disease of cMSC1 would be further enhanced. An *in vitro* experiment was performed to identify the enhancement in therapeutic efficacy for graft-versus-host disease. The inhibition rate of activated T cells was identified under mixed lymphocyte reaction (MLR) conditions, and regarding the expression levels of immune-related markers (IDO, ICOSL, TSG6, CXCL9, CXCL10, and HO-1) in the state of cells without any stimulation, the two cells were compared by a qPCR method. The results are shown in FIG. 44.

As shown in FIG. 44, as a result of identifying the inhibition rate of activated T cells under mixed lymphocyte reaction conditions, both cells showed a high inhibition rate of 95% under the condition of T cell : MSC = 1 : 4. However, as a result of comparing the expression levels of immune-related markers (IDO, ICOSL, TSG6, CXCL9, CXCL10, and HO-1) in a state without any stimulation, it was identified that the expression level of the immune-related markers of cMSC1 was high. This is a result showing that cMSC1 may exhibit better therapeutic efficacy than cells obtained through conventional methods when used as a therapeutic agent for graft-versus-host disease.

### 5.2. Comparison of GVHD Therapeutic Effect of Stem Cells obtained through Improved Subfractionation Culturing Method and Stem Cells obtained through Conventional Subfractionation Culturing Method

Since it was identified that the stem cells obtained through the improved subfractionation culturing method through Example 5.1 exhibited more suitable cell characteristics as a GVHD therapeutic agent compared to the stem cells obtained through the conventional subfractionation culturing method, an acute GVHD animal model was prepared, and the therapeutic effects of cMSC1 and cMSC2 were identified from an animal model.

In order to remove all the immune cells of the receptor BALB/c to establish an acute GVHD model, the irradiation of 7.0 Gy, close to lethal dose, was irradiated, and each of the bone marrow cells and splenocytes isolated from the recipient C57BL/6N after about 24 hours was mixed with of 5 × 10⁶/100 µl of PBS, and 200 µl was injected into the irradiated BALB/c through the caudal vein to induce disease. Thereafter, stem cells cMSC1 and cMSC2 obtained through an improved subfractionation culturing method were administered to the established GVHD animal model. Stem cells obtained by subculture at 100 cells/cm² (cMSC1 (100)) and 1,000 cells/cm² (cMSC1 (1000)) were used as a stem cell experimental group obtained by the improved subfractionation culturing method culturing cells at 1,000 cells/cm² or less. The high-density culture cMSC2 corresponding to the conventional subfractionation culturing method used stem cells obtained by subculture at 4,000 cells/cm². In all of the cMSC1 (100), cMSC1 (1000), and cMSC2 experimental groups, the cell survival rate averaged 99.53%, 99.66%, and 99.8%, respectively, identifying that the density regulation culture did not show cytotoxicity. The administered substances and total dosage administered to the cell cGVHD animal model are shown in Table 12 below.

**[Table 12]**

| Group | Inducing substance | Administered substance | Total dosage (cells/head) | Total amount of administered solution (µl/head) |
|---|---|---|---|---|
| G1 | - | - | - | - |
| G2 | Allogeneic Bone marrow | - | - | - |
| G3 | Allogeneic splenocyte | PBS | 1.0×10⁶ | 600µl |
| G4 | Allogeneic splenocyte | cMSC1 (100) | 1.0×10⁶ | 600µl |
| G5 | Allogeneic splenocyte | cMSC1 (1000) | 1.0×10⁶ | 600µl |
| G6 | Allogeneic splenocyte | cMSC2 (4000) | 1.0×10⁶ | 600µl |

The severity of GVHD was identified by scoring the severity of each symptom, such as dead skin cells and wounds, hair loss and roughness observed visually on the skin, hunched back, diarrhea and ascites, posture, and movement, and analyzed according to survival rate.

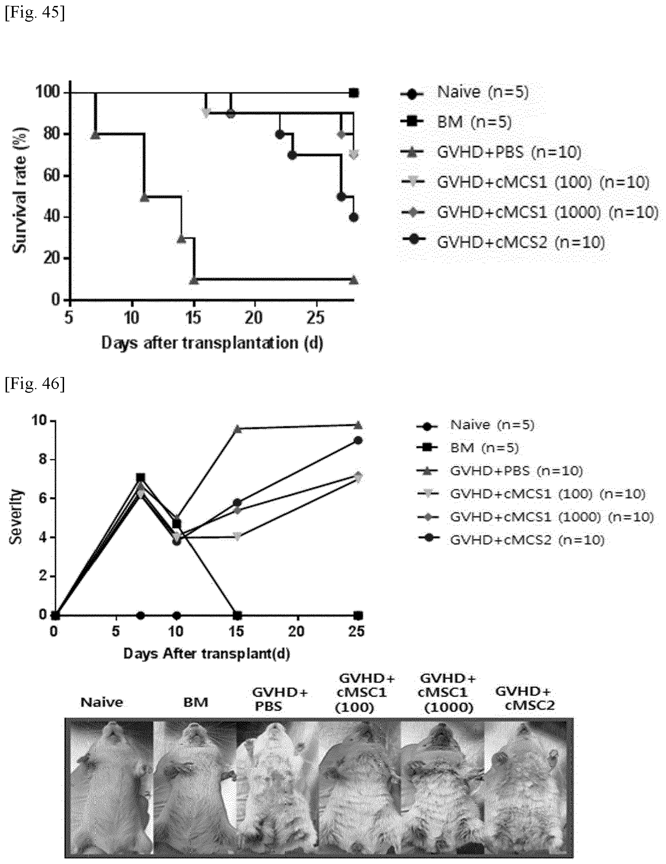

The results of identifying the survival rate according to the administration of each experimental group are shown in FIG. 45 and Table 13.

**[Table 13]**

| Time after induction (days) | | **7** | **11** | **14** | **15** | **18** | **23** | **27** | **28** |
|---|---|---|---|---|---|---|---|---|---|
| Survival rate **(%)** | **Naïve (G1)** | 100 | 100 | 100 | 100 | 100 | 100 | 100 | 100 |
| | **BM (G2)** | 100 | 100 | 100 | 100 | 100 | 100 | 100 | 100 |
| | **PBS(G3)** | 80 | 50 | 30 | 10 | 10 | 10 | 10 | 10 |
| | **cMSC1 (100) (G4)** | 100 | 100 | 100 | 100 | 90 | 90 | 90 | 70 |
| | **cMSC1 (1000) (G5)** | 100 | 100 | 100 | 100 | 90 | 90 | 80 | 70 |
| | **cMSC2 (4000) (G6)** | 100 | 100 | 100 | 100 | 90 | 70 | 50 | 40 |

As shown in Table 13 and FIG. 45, in the normal control group (Naive, G1) and the group transplanted only with bone marrow cells (BM, G2), all subjects survived during the 28-day observation period. However, in the experimental group in which GVHD was induced and only PBS was administered (GVHD + PBS, G3), it was identified that the survival rate dropped to 80% or less from the 7th day, 50% on the 11th day, and all subjects died except 1 mouse on the 15th day.

On the other hand, irrespective of the culture conditions, it was identified that the time point at which the survival rate fell in both cMSC-injected groups increased by two or more times when compared to the PBS-only injection group (PBS: 7 days, cMSC injection group: 16 to 18 days). Specifically, in the group administered with stem cells (cMSC2, G6) obtained by the conventional subfractionation culturing method, the survival rate began to decrease from day 18, and the survival rate decreased up to 50% on day 27, and 40% of the total survived until the observation period (day 28). It was identified that the survival rate of both administration groups administered with cMSC1 (100) and cMSC1 (1000) obtained through the improved subfractionation culturing method decreased from day 18, but the survival rate was maintained thereafter, and that 70% of the total survived until day 28. This is a result showing that with an increase in survival rate of about 1.5 times or more compared to the conventional subfractionation culturing method, the stem cells obtained through the improved subfractionation culturing method may exhibit an excellent GVHD therapeutic effect compared to the stem cells obtained through the conventional sub fractionation culturing method.

The results of evaluating the clinical symptoms of the GVHD animal model according to the experimental group are shown in FIG. 46.

As shown in FIG. 46, after 28 days of stem cell transplantation, as a result of observing clinical symptoms comprehensively evaluating dead skin cells and bloodstain, hair condition, ascites, diarrhea, hunched back, and the like, it was identified that the clinical symptoms were improved in the cMSC injection group regardless of the culture conditions compared to GVHD + PBS (G3) group. In particular, in GVHD + cMSC1 (100) (G4) and GVHD + cMSC1 (1000) (G5), more effective clinical symptom alleviation was identified compared to the high-density cultured cMSC2 administration group (G6) obtained by the conventional subfractionation culturing method. This difference was evident from day 15 onwards. According to the condition of the experimental animals on the 28th day, it was identified visually that the clinical symptoms of the GVHD + cMSC1 (100) (G4) and GVHD + cMSC1 (1000) (G5) experimental groups were improved compared to the PBS and cMSC2 administration groups.

To sum up the above results, it was identified that the use of cMSC1 obtained through the improved subfractionation culturing method may increase the survival rate of subjects with graft-versus-host disease and effectively alleviate various clinical symptoms. In particular, cMSC1 according to the present disclosure is capable of maintaining excellent proliferation ability and stem cell characteristics compared to the conventional monoclonal mesenchymal stem cells to effectively maintain proliferation, as well as omitting the production process of WCB compared to the monoclonal mesenchymal stem cells produced for the production of existing therapeutic agents and being obtained in a small number of passages, and thus is a single colony in a fresh state without aging. Therefore, cMSC1 produces a more excellent therapeutic effect on graft-versus-host disease than the conventional monoclonal mesenchymal stem cells.

## Claims

1. A pharmaceutical composition for use in preventing or treating a graft-versus-host disease, the pharmaceutical composition comprising monoclonal stem cells obtained by steps of
1) culturing bone marrow isolated from a subject in a first vessel;
2) transferring only a supernatant from the first vessel to a new vessel and culturing the supernatant;
3) culturing cells present in the new vessel and obtaining a supernatant;
4) obtaining the monoclonal stem cells by repeating steps 2) and 3) at least once using the supernatant of step 3) as the supernatant of the first vessel of step 2);
5) inoculating the monoclonal stem cells of step 4) in a medium at a cell density of 50 to 1,000 cells/cm² to culture the cells at passage 1; and
6) inoculating the subcultured monoclonal stem cells of step 5) in a medium at a cell density of 50 to 1000 cells/cm² to culture the cells at passages 2 to 13, wherein the medium of step 5) and 6) is supplemented with an antioxidant.

2. The composition for use according to claim 1, wherein the culture of step 5) or step 6) is performed by inoculating the monoclonal stem cells in a medium at a cell density of 1,000 cells/cm².

3. The composition for use according to claim 1, wherein the graft-versus-host disease is an acute graft-versus-host disease or a chronic graft-versus-host disease.

4. The composition for use according to claim 1, wherein the monoclonal stem cells have increased expression levels of one or more immune-related markers selected from the group consisting of IDO (Indoleamine 2,3-dioxygenase), ICOSL (Induced T cell co-stimulator ligand), TSG6 (Tumor necrosis factor-inducible gene 6 protein), CXCL9 (chemokine (C-X-C motif) ligand 9), CXCL10 (chemokine (C-X-C motif) ligand 10), and HO-1 (Heme oxygenase-1).

5. A method of producing a pharmaceutical composition comprising monoclonal stem cells, the method comprising steps of:
1) culturing bone marrow isolated from a subject in a first vessel;
2) transferring only a supernatant from the first vessel to a new vessel and culturing the supernatant;
3) culturing cells present in the new vessel and obtaining a supernatant;
4) obtaining monoclonal stem cells by repeating steps 2) and 3) at least once using the supernatant of step 3) as the supernatant of the first vessel of step 2);
5) inoculating the monoclonal stem cells of step 4) in a medium at a cell density of 50 to 1,000 cells/cm² to culture the cells at passage 1; and
6) inoculating the subcultured monoclonal stem cells of step 5) in a medium at a cell density of 50 to 1000 cells/cm² to culture the cells at passages 2 to 13, wherein the medium of step 5) and 6) is supplemented with an antioxidant.

6. The method of claim 5, wherein the culture of step 5) or 6) is performed by inoculating the monoclonal stem cells in a medium at a cell density of 1,000 cells/cm².

## Patentansprüche

1. Pharmazeutische Zusammensetzung zur Verwendung bei der Vorbeugung oder Behandlung einer Graft-versus-Host-Krankheit, wobei die pharmazeutische Zusammensetzung monoklonale Stammzellen umfasst, die mit folgenden Schritten erhalten werden:
1) Kultivieren von einem Individuum entnommenem Knochenmark in einem ersten Gefäß;
2) Überführen nur eines Überstands aus dem ersten Gefäß in ein neues Gefäß und Kultivieren des Überstands;
3) Kultivieren von im neuen Gefäß vorhandenen Zellen und Gewinnen eines Überstands;
4) Gewinnen der monoklonalen Stammzellen durch wenigstens einmaliges Wiederholen der Schritte 2) and 3) unter Verwendung des Überstands aus Schritt 3) als Überstand aus dem ersten Gefäß in Schritt 2);
5) Animpfen eines Mediums mit den monoklonalen Stammzellen aus Schritt 4) bei einer Zelldichte von 50 bis 1.000 Zellen/cm² zum Kultivieren der Zellen bei Passagierung 1; und
6) Animpfen eines Mediums mit den subkultivierten monoklonalen Stammzellen aus Schritt 5) bei einer Zelldichte von 50 bis 1000 Zellen/cm² zum Kultivieren der Zellen bei Passagierung 2 bis 13,
wobei das Medium in Schritt 5) und 6) mit einem Antioxidationsmittel supplementiert ist.

2. Zusammensetzung zur Verwendung nach Anspruch 1, wobei die Kultur in Schritt 5) oder Schritt 6) durchgeführt wird, indem ein Medium mit den monoklonalen Stammzellen bei einer Zelldichte von 1.000 Zellen/cm² angeimpft wird.

3. Zusammensetzung zur Verwendung nach Anspruch 1, wobei es sich bei der Graft-versus-Host-Krankheit um eine akute Graft-versus-Host-Krankheit oder eine chronische Graft-versus-Host-Krankheit handelt.

4. Zusammensetzung zur Verwendung nach Anspruch 1, wobei die monoklonalen Stammzellen erhöhte Expressionsniveaus eines oder mehrerer immunverwandter Marker aufweisen, die aus der Gruppe bestehend aus IDO (Indolamin-2,3-Dioxygenase), ICOSL (Induzierter T-Zell-Costimulator-Ligand), TSG6 (Tumornekrosefaktor-induzierbares-Gen-6-Protein), CXCL9 (Chemokin-(C-X-C-Motiv)-Ligand 9), CXCL10 (Chemokin-(C-X-C motif)-Ligand 10) und HO-I (Häm-Oxygenase-1) ausgewählt sind.

5. Verfahren zur Herstellung einer monoklonale Stammzellen umfassenden pharmazeutischen Zusammensetzung, wobei das Verfahren folgende Schritte umfasst:
1) Kultivieren von einem Individuum entnommenem Knochenmark in einem ersten Gefäß;
2) Überführen nur eines Überstands aus dem ersten Gefäß in ein neues Gefäß und Kultivieren des Überstands;
3) Kultivieren von im neuen Gefäß vorhandenen Zellen und Gewinnen eines Überstands;
4) Gewinnen monoklonaler Stammzellen durch wenigstens einmaliges Wiederholen der Schritte 2) and 3) unter Verwendung des Überstands aus Schritt 3) als Überstand aus dem ersten Gefäß in Schritt 2);
5) Animpfen eines Mediums mit den monoklonalen Stammzellen aus Schritt 4) bei einer Zelldichte von 50 bis 1.000 Zellen/cm² zum Kultivieren der Zellen bei Passagierung 1; und
6) Animpfen eines Mediums mit den subkultivierten monoklonalen Stammzellen aus Schritt 5) bei einer Zelldichte von 50 bis 1000 Zellen/cm² zum Kultivieren der Zellen bei Passagierung 2 bis 13,
wobei das Medium in Schritt 5) und 6) mit einem Antioxidationsmittel supplementiert ist.

6. Verfahren gemäß Anspruch 5, wobei die Kultur in Schritt 5) oder Schritt 6) durchgeführt wird, indem ein Medium mit den monoklonalen Stammzellen bei einer Zelldichte von 1.000 Zellen/cm² angeimpft wird.

## Revendications

1. Composition pharmaceutique pour une utilisation dans la prévention ou le traitement d'une maladie du greffon contre l'hôte, la composition pharmaceutique comprenant des cellules souches monoclonales par des étapes de :
1) culture de moelle osseuse isolée d'un sujet dans un premier récipient ;
2) transfert seulement du surnageant du premier récipient à un nouveau récipient et culture du surnageant ;
3) culture de cellules présentes dans le nouveau récipient et obtention d'un surnageant ;
4) obtention des cellules souches monoclonales par répétition des étapes 2) et 3) au moins une fois en utilisant le surnageant de l'étape 3) comme surnageant du premier récipient de l'étape 2) ;
5) inoculation des cellules souches monoclonales de l'étape 4) dans un milieu à une densité de cellules de 50 à 1 000 cellules/cm² pour cultiver les cellules au passage 1 ; et
6) inoculation des cellules souches monoclonales sous-cultivées de l'étape 5) dans un milieu à une densité de cellules de 50 à 1 000 cellules/cm² pour cultiver les cellules aux passages 2 à 13,
le milieu de l'étape 5) et 6) étant supplémenté avec un antioxydant.

2. Composition pour une utilisation selon la revendication 1, la culture de l'étape 5) ou de l'étape 6) étant réalisée par inoculation des cellules souches monoclonales dans un milieu à une densité de cellules de 1 000 cellules/cm².

3. Composition pour une utilisation selon la revendication 1, la maladie du greffon contre l'hôte étant une maladie du greffon contre l'hôte aigüe ou une maladie du greffon contre l'hôte chronique.

4. Composition pour une utilisation selon la revendication 1, les cellules souches monoclonales ayant des niveaux d'expression augmentés d'un ou plusieurs marqueurs liés au système immunitaire choisis dans le groupe constitué par IDO (indolamine 2,3-dioxygénase), ICOSL (ligand co-stimulateur de cellules T induit), TSG6 (protéine du gène 6 inductible par le facteur de nécrose tumorale), CXCL9 (ligand 9 de chimiokine (motif C-X-C)), CXCL10 (chimiokine (motif C-X-C)) ligand 10), et HO-I (hème oxygénase-1).

5. Procédé de production d'une composition pharmaceutique comprenant des cellules souches monoclonales, le procédé comprenant les étapes de :
1) culture de moelle osseuse isolée d'un sujet dans un premier récipient ;
2) transfert seulement du surnageant du premier récipient à un nouveau récipient et culture du surnageant ;
3) culture de cellules présentes dans le nouveau récipient et obtention d'un surnageant ;
4) obtention des cellules souches monoclonales par répétition des étapes 2) et 3) au moins une fois en utilisant le surnageant de l'étape 3) comme surnageant du premier récipient de l'étape 2) ;
5) inoculation des cellules souches monoclonales de l'étape 4) dans un milieu à une densité de cellules de 50 à 1 000 cellules/cm² pour cultiver les cellules au passage 1 ; et
6) inoculation des cellules souches monoclonales sous-cultivées de l'étape 5) dans un milieu à une densité de cellules de 50 à 1 000 cellules/cm² pour cultiver les cellules aux passages 2 à 13,
le milieu de l'étape 5) et 6) étant supplémenté avec un antioxydant.

6. Procédé selon la revendication 5, la culture de l'étape 5) ou de l'étape 6) étant réalisée par inoculation des cellules souches monoclonales dans un milieu à une densité de cellules de 1 000 cellules/cm².
